# DEMANDE DE BREVET EUROPEEN

(11) **EP 1 619 220 A1**
(43) Date de publication de la demande: **25.01.2006**
(21) Numéro de dépôt: 05291349.8
(22) Date de dépôt: 23.06.2005
(51) Int. Cl.: C09B 44/16, C09B 44/12, C07D 401/14

(54) **Composes diazoiques cationiques particuliers, compositions les comprenant à titre de colorant direct, procédé de coloration de fibres kératiniques et dispositif**

(30) Priorité: 23.06.2004 FR 0406870
(71) Demandeur: L'Oreal-D.I.P.I., 75008 Paris (FR)
(72) Inventeur: Greaves, Andrew, 77144 Montevrain (FR); David, Hervé, 94340 Joinville le Pont (FR)
(74) Mandataire: Wattremez, Catherine

(57) **Abrégé**

La présente invention a pour objet des composés cationiques diazoïques de formules suivantes :
Col1 - LK - Col2

Dans laquelle :
Col1 et Col2 représentent :

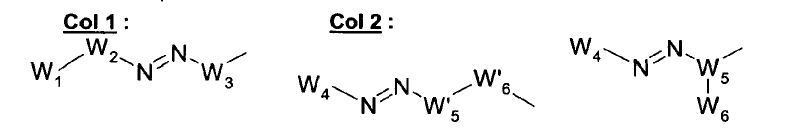

Dans lesquelles
W₁, W₆:-NR₁R₂, -OR₃ / W₆ : -NR'₁-
W₂, W₅, W'₅: groupement de formules (a) à (c) suivantes : 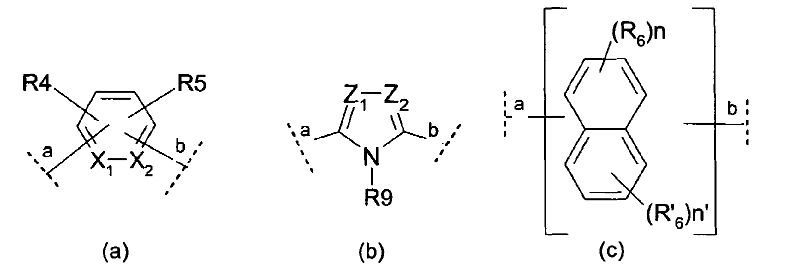
W₃, W₄ : hétérocycle aromatique cationique ;
LK : chaîne hydrocarbonée en C₁-C₄₀, saturée ou non, linéaire ou ramifiée, cyclique ou non, aromatique ou non, éventuellement substituée, éventuellement interrompue par au moins un hétéroatome ou groupement en comprenant au moins un.

Elle a de plus pour objet des compositions tinctoriales comprenant lesdits composés à titre de colorant direct, ainsi qu'un procédé de coloration de fibres kératiniques mettant en oeuvre cette composition et un dispositif à plusieurs compartiments.

## Description

La présente invention a pour objet des composés diazoïques cationiques particuliers, des compositions tinctoriales comprenant dans un milieu approprié pour la teinture des fibres kératiniques, de tels composés à titre de colorant direct, ainsi qu'un procédé de coloration de fibres kératiniques mettant en oeuvre cette composition et un dispositif à plusieurs compartiments.

Il est connu de teindre les fibres kératiniques et en particulier les fibres kératiniques humaines, telles que les cheveux, avec des compositions tinctoriales contenant des colorants directs. Ces composés sont des molécules colorées et colorantes ayant une affinité pour les fibres. Il est connu par exemple d'utiliser des colorants directs du type nitrés benzéniques, des colorants anthraquinoniques, des nitropyridines, des colorants du type azoïque, xanthénique, acridinique, azinique ou triarylméthane.

Habituellement, ces colorants sont appliqués sur les fibres, éventuellement en présence d'un agent oxydant si l'on souhaite obtenir un effet simultané d'éclaircissement des fibres. Une fois le temps de pose écoulé, les fibres sont rincées, éventuellement lavées et séchées.

Les colorations qui résultent de l'utilisation de colorants directs sont des colorations temporaires ou semi-permanentes car la nature des interactions qui lient les colorants directs à la fibre kératinique, et leur désorption de la surface et/ou du coeur de la fibre sont responsables de leur faible puissance tinctoriale et de leur relative mauvaise tenue aux lavages ou à la transpiration.

On est aussi confronté à une difficulté supplémentaire, liée au fait que pour obtenir une couleur particulière, il est nécessaire dans la majeure partie des cas, pour ne pas dire la totalité, de mélanger plusieurs colorants. Or chaque colorant ne possède pas la même affinité pour la fibre, ce qui se traduit soit par des colorations hétérogènes, soit par des virages de couleur au cours du temps, par exemple après un ou plusieurs lavages des fibres, exposition au soleil, etc.

Le but de la présente invention est de fournir des colorants directs ne présentant pas les inconvénients des colorants directs existants.

En particulier, l'un des buts de la présente invention est de fournir des colorants directs qui permettent d'obtenir des nuances variées sans problème de virage de la couleur dans le temps.

Ces buts et d'autres sont atteints par la présente invention qui a donc pour objet des nouveaux composés diazoïques cationiques de formule (I) suivante ou leurs sels d'addition avec un acide :

Col1 - LK - Col2 (I)

Dans laquelle Col1 et Col2 représentent : 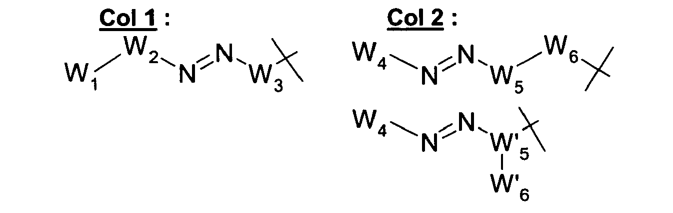
Dans lesquelles
**W**_{**1**} **et W'**_{**6**} indépendamment l'un de l'autre représentent un groupement -NR₁R₂, -OR₃ dans lesquels R₁, R₂ et R₃ indépendamment l'un de l'autre représentent un atome d'hydrogène, une chaîne hydrocarbonée en C₁-C₂₀, de préférence en C₁-C₁₆, saturée ou insaturée, aromatique ou non, pouvant former un ou plusieurs cycles carbonés éventuellement substitués, comportant de 3 à 7 chaînons, éventuellement substituée, éventuellement interrompue par un ou plusieurs hétéroatomes ou par un ou plusieurs groupements portant au moins un hétéroatome, de préférence choisi parmi l'oxygène, l'azote ; R₁ et R₂ pouvant éventuellement former avec l'atome d'azote auquel ils sont rattachés, un hétérocycle à 5 à 7 chaînons, éventuellement substitué et comprenant éventuellement un autre hétéroatome identique ou différent de l'azote ;
Les radicaux R₁, R₂, R₃ issus de W'₆ indépendamment les uns des autres peuvent éventuellement former avec une partie du groupement LK et avec l'atome d'azote ou d'oxygène auquel chacun est rattaché, un hétérocycle à 5 ou 7 chaînons, saturé ou non, aromatique ou non éventuellement substitué ;
**W**_{**6**}, représente un groupement -NR'₁-, -O- dans lequel R'₁, représente un atome d'hydrogène, une chaîne hydrocarbonée en C₁-C₂₀, de préférence en C₁-C₁₆, saturée ou insaturée, aromatique ou non, pouvant former un ou plusieurs cycles carbonés éventuellement substitués, comportant de 3 à 7 chaînons, éventuellement substituée, éventuellement interrompue par un ou plusieurs hétéroatomes ou par un ou plusieurs groupements portant au moins un hétéroatome, de préférence choisi parmi l'oxygène, l'azote ;
Le radical R'₁ issu de W₆ peut éventuellement former avec l'atome d'azote auquel il est rattaché et une partie du groupement LK, un hétérocycle comprenant de 5 à 7 chaînons, saturé ou non, aromatique ou non comprenant éventuellement un autre hétéroatome, choisi parmi l'azote, l'oxygène, et pouvant être éventuellement substitué ;
**W**_{**2**}**, W**_{**5**} **et W'**_{**5**}**,** indépendamment les uns des autres, représentent un groupement de formules (a) à (c) suivantes : 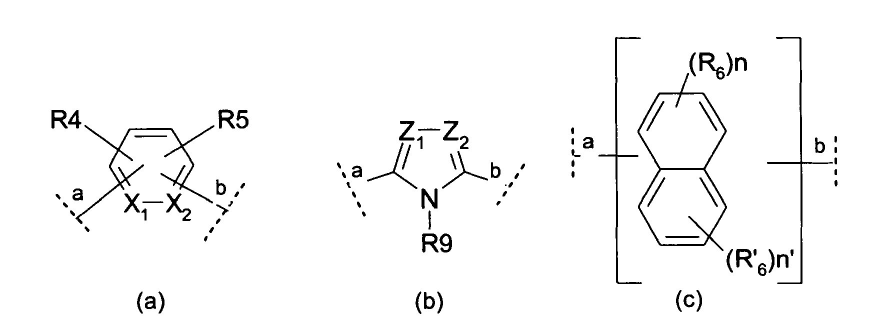
Formules dans lesquelles :
- X₁ représente un atome d'azote ou un groupement CR₇ ;
- X₂ représente un atome d'azote ou un groupement CR₈ ;
- Z₁ représente un atome d'azote ou un groupement CR₁₀ ;
- Z₂ représente un atome d'azote ou un groupement CR₁₁ ;
- R₄, R₅, R₆, R'₆, R₇, R₈, R₁₀, R₁₁, indépendamment les uns des autres, représentent :
   - une chaîne hydrocarbonée en C₁-C₁₆, linéaire ou ramifiée, saturée ou insaturée, aromatique ou non, pouvant former un ou plusieurs cycles carbonés comportant de 3 à 6 chaînons, éventuellement substituée, éventuellement interrompue par un ou plusieurs hétéroatomes ou par un ou plusieurs groupements portant au moins un hétéroatome, de préférence choisi parmi l'oxygène, l'azote ;
   - un groupement hydroxyle,
   - un groupement alcoxy en C₁-C₄, un groupement (poly)hydroxyalcoxy en C₂-C₄ ; un groupement alcoxycarbonyle (RO-CO-) dans lequel R représente un radical alkyle en C₁-C₄, un radical alkylcarbonyloxy (RCO-O-) dans lequel R représente un radical alkyle est en C₁-C₄ ;
   - un groupement amino, un groupement amino substitué par un ou plusieurs radicaux alkyles en C₁-C₄, identiques ou différents, éventuellement porteurs d'au moins un groupement hydroxyle, les deux radicaux alkyle pouvant éventuellement former avec l'atome d'azote auquel ils sont rattachés, un hétérocycle à 5 ou 6 chaînons éventuellement porteur d'un autre hétéroatome identique ou différent de l'azote ; un groupement alkylcarbonylamino (RCO-NR-) dans lequel les radicaux R indépendamment les uns des autres représentent un radical alkyle en C₁-C₄; un groupement carbamoyle ((R)₂N-CO) dans lequel les radicaux R indépendamment les uns des autres , identiques ou non, représentent un atome d'hydrogène, un radical alkyle en C₁-C₄ ; un groupement uréido (N(R)₂-CO-NR'-) dans lequel les radicaux R et R' , indépendamment les uns des autres, représentent un atome d'hydrogène, un radical alkyle en C₁-C₄ ; un groupement sulfonamide ((R)₂N-SO₂-) dans lequel les radicaux R, indépendamment les uns des autres, représentent un atome d'hydrogène, un radical alkyle en C₁-C₄, ; un groupement alkylsulfonylamino (RSO₂-NR'-) dans lequel les radicaux R, R', indépendamment les uns des autres, représentent un atome d'hydrogène, un radical alkyle en C₁-C₄; un groupement guanidinium ((R')₂N-C(=NH₂⁺)-NR-) dans lequel les radicaux R et R', indépendamment les uns des autres, représentent un atome d'hydrogène, un radical alkyle en C₁-C₄ ;

   - un groupement nitro ; un groupement cyano ; un atome d'halogène, de préférence le chlore, le fluor ;
   - R₄, R₅, R₆, R'₆, R₇, R₈, R₁₀, R₁₁ peuvent représenter un atome d'hydrogène ;
   - R₄, R₅, R₆, R'₆, R₇, R₈, R₁₀, R₁₁, indépendamment les uns des autres, peuvent éventuellement former avec tout ou partie des groupements W'₁ ou W'₆ un hétérocycle comprenant de 5 à 7 chaînons, saturé ou non, éventuellement substitué ;

   - une liaison de W'₅ à W'₆, ou au groupement LK ;
   - a représente la liaison de W₂, W₅, W'₅ au groupement azoïque -N=N- ;
   - b représente la liaison de W₂ à W₁, de W₅ à W₆, de W'₅ à W'₆ ;
- R₉ représente :
   - un atome d'hydrogène,
   - une chaîne hydrocarbonée en C₁-C₁₆, linéaire ou ramifiée, saturée ou insaturée, aromatique ou non, pouvant former un ou plusieurs cycles carbonés comportant de 3 à 7 chaînons, éventuellement substituée,
- n et n' représentent des nombres entiers et leur somme est inférieure ou égale à 6 ; il est à noter que lorsque la somme de n et n' est inférieure à 6, chaque substituant maquant est un atome d'hydrogène ;

**W**_{**3**}**, W**_{**4**}**,** représentent, indépendamment l'un de l'autre, un radical hétéroaromatique cationique représentés par l'une des formules (1) et (11) suivantes :

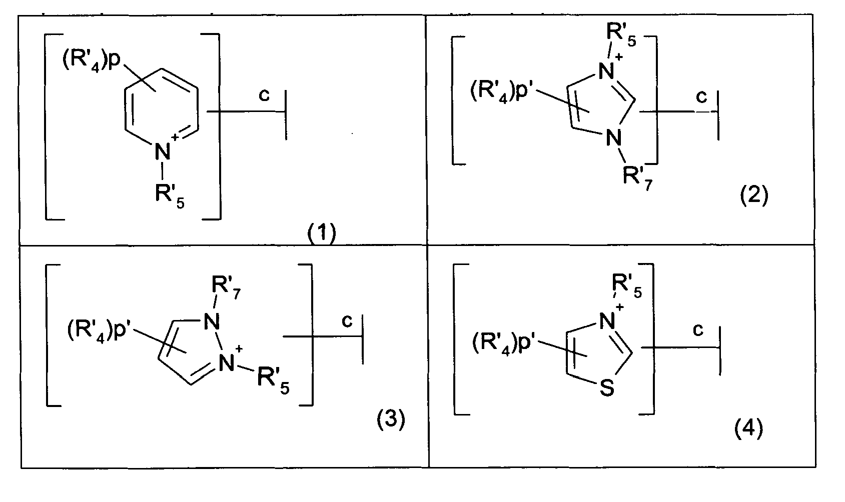

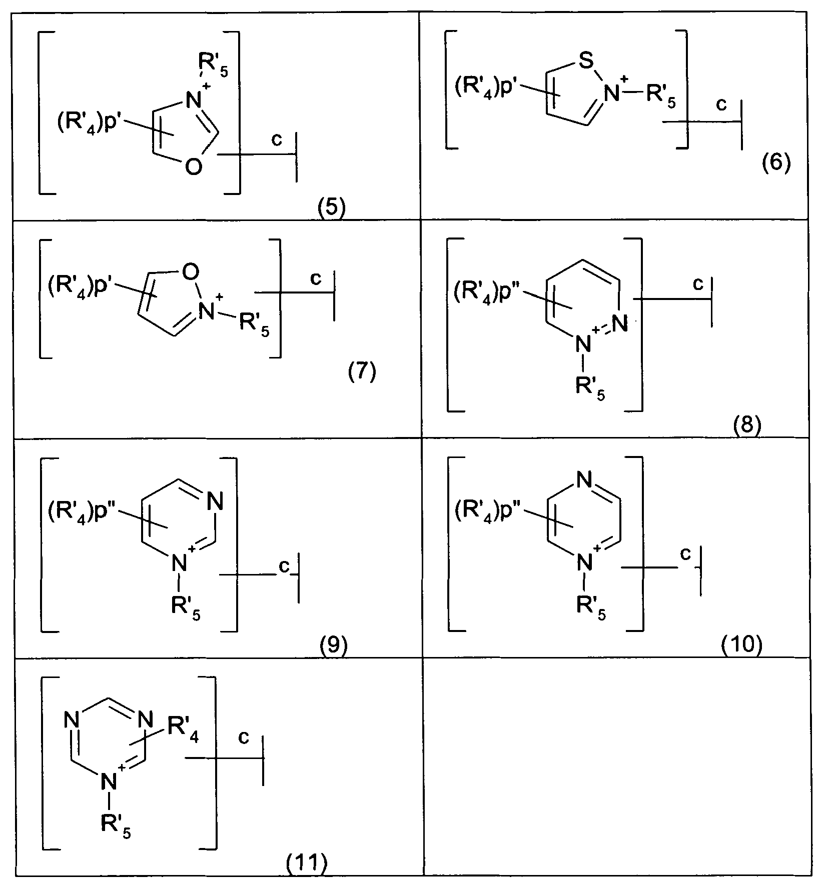

Dans lesquelles :
- R'₄ identiques ou différents, substituant le cycle principal, représentent :
   - une chaîne hydrocarbonée en C₁-C₁₆, linéaire ou ramifiée, saturée ou insaturée, aromatique ou non, pouvant former un ou plusieurs cycles carbonés comportant de 3 à 6 chaînons, éventuellement substituée, éventuellement interrompue par un ou plusieurs hétéroatomes ou par un ou plusieurs groupements portant au moins un hétéroatome, de préférence choisi parmi l'oxygène, l'azote ;
   - un groupement hydroxyle,
   - un groupement alcoxy en C₁-C₄, un groupement (poly)hydroxyalcoxy en C₂-C₄ ; un groupement alcoxycarbonyle (RO-CO-) dans lequel R représente un radical alkyle en C₁-C₄, un radical alkylcarbonyloxy (RCO-O-) dans lequel R représente un radical alkyle est en C₁-C₄ ;
   - un groupement amino, un groupement amino substitué par un ou plusieurs radicaux alkyles en C₁-C₄, indépendamment les uns des autres, éventuellement porteurs d'au moins un groupement hydroxyle, les deux radicaux alkyle pouvant éventuellement former avec l'atome d'azote auquel ils sont rattachés, un hétérocycle à 5 ou 6 chaînons éventuellement porteur d'un autre hétéroatome identique ou différent de l'azote ; un groupement alkylcarbonylamino (RCO-NR'-) dans lequel le radical R représente un radical alkyle en C₁-C₄ et le radical R' représente un hydrogène, un radical alkyle en C₁-C₄ ; un groupement carbamoyle ((R)₂N-CO-) dans lequel les radicaux R indépendamment les uns des autres, représentent un atome d'hydrogène, un radical alkyle en C₁-C₄ ; un groupement uréido (N(R)₂-CO-NR'-) dans lequel les radicaux R et R', indépendamment les uns des autres, représentent un atome d'hydrogène, un radical alkyle en C₁-C₄ ; un groupement sulfonamide ((R)₂N-SO₂-) dans lequel les radicaux R indépendamment les uns des autres, représentent un atome d'hydrogène, un radical alkyle en C₁-C₄ ; un groupement alkylsulfonylamino (RSO₂-NR'-) dans lequel les radicaux R, R', indépendamment les uns des autres, représentent un atome d'hydrogène, un radical alkyle en C₁-C₄ ; un groupement guanidinium ((R')₂N-C(=NH₂⁺)-NR-) dans lequel les radicaux R et R', indépendamment les uns des autres, représentent un atome d'hydrogène, un radical alkyle en C₁-C₄ ;
   - un groupement nitro ; un groupement cyano ; un atome d'halogène, de préférence le chlore, le fluor ;
   - deux radicaux R'₄ portés par deux atomes de carbone adjacents du cycle principal peuvent éventuellement former un cycle secondaire, aromatique ou non, à 5 ou 6 chaînons, éventuellement substitué par au moins un hydrogène ; au moins un groupement hydroxyle ; au moins un radical alkyle en C₁-C₄ ; au moins un radical alcoxy en C₁-C₄ ; au moins un radical (poly)hydroxyalcoxy en C₂-C₄ ; au moins un radical amino ; au moins un radical amino substitué par un ou plusieurs radicaux identiques ou différents, alkyle en C₁-C₄, éventuellement porteurs d'au moins un groupement hydroxyle ; de préférence, le cycle secondaire est un cycle aromatique à 6 chaînons éventuellement substitué comme indiqué précédemment ;
- R'₅ porté par l'atome d'azote quaternisé, dans le cas de W₄, représente une chaîne hydrocarbonée en C₁-C₁₆, linéaire ou ramifiée, saturée ou insaturée, aromatique ou non, pouvant former un ou plusieurs cycles carbonés éventuellement substitués comportant de 3 à 6 chaînons, éventuellement substituée, éventuellement interrompue par un ou plusieurs hétéroatomes ou par un ou plusieurs groupements portant au moins un hétéroatome, de préférence choisi parmi l'oxygène, l'azote ; le radical R'₅ est tel que l'atome directement relié à l'atome d'azote quaternisé est un atome de carbone ;
- R'₅ porté par l'atome d'azote quaternisé, dans le cas de W₃, représente une liaison à LK ;
- R'₇ représente un radical alkyle en C₁-C₄ éventuellement substitué ; un radical phényle éventuellement substitué ; un radical benzyle éventuellement substitué ;
- La liaison c relie le radical cationique défini par les formules (1) à (11) au groupement azoïque ; ladite liaison pouvant se trouver sur le cycle principal ou secondaire ; de préférence, la liaison a avec le groupement azoïque se trouve sur le cycle principal ;
- p est un nombre entier variant de 0 à 4, p' est un nombre entier variant de 0 à 2 et p" est un nombre entier variant de 0 à 3 ; il est à noter que lorsque le cycle principal ne porte pas le nombre de substituant maximum, alors la position non substituée porte un atome d'hydrogène ;

**LK** représente une chaîne hydrocarbonée en C₂-C₄₀, de préférence en C₂-C₂₀, saturée ou non, linéaire ou ramifiée, cyclique ou non, aromatique ou non, éventuellement substituée, éventuellement interrompue par au moins un hétéroatome ou groupement comprenant au moins un hétéroatome, de préférence l'oxygène, l'azote ; le groupement LK ne comporte par ailleurs pas de liaison ou groupement peroxo, nitro, nitroso ; si LK est relié à W'₅, LK peut être terminé par un hétéroatome ou groupement comprenant au moins un hétéroatome, de préférence l'oxygène, l'azote ; si LK est relié à W₆, LK peut être terminé par un groupement comprenant au moins un hétéroatome choisi parmi -CO-, -SO₂- ; si LK est relié à W₃, la liaison se fait par l'intermédiaire d'un atome de carbone ;
L'électroneutralité des composés étant assurée par un ou plusieurs anions An cosmétiquement acceptables.

La présente invention a de même pour objet des compositions tinctoriales comprenant, dans un milieu approprié pour la teinture des fibres kératiniques, de tels composés, ou ses sels d'addition avec un acide, à titre de colorant direct.

Elle concerne de plus un procédé de coloration de fibres kératiniques consistant à mettre en contact une telle composition, avec les fibres sèches ou humides, pendant une durée suffisante pour obtenir l'effet souhaité.

Elle a enfin pour objet un dispositif à plusieurs compartiments comprenant, dans un premier compartiment, la composition selon l'invention, et dans un second compartiment, une composition oxydante.

On a constaté que les composés de formule (I) présentaient une bonne ténacité vis-à-vis d'agents extérieurs comme notamment les shampooings, et cela, même lorsque la fibre kératinique est sensibilisée.

De plus, les composés, qui sont des composés dissymétriques, permettent d'accéder à des colorations qui sont moins chromatiques par rapport à des composés symétriques.

Mais d'autres caractéristiques et avantages de la présente invention apparaîtront plus clairement à la lecture de la description et des exemples qui vont être présentés.

Dans ce qui va suivre et à moins qu'une autre indication ne soit donnée, les bornes délimitant un domaine de valeurs sont comprises dans ce domaine.

Par ailleurs, les fibres kératiniques faisant l'objet du traitement selon l'invention sont des fibres kératiniques humaines, en particulier les cheveux.

Au sens de la présente invention, et à moins qu'une indication différente ne soit donnée :
- Un radical alkyle est linéaire ou ramifié,
- Un radical alkyle ou la partie alkyle d'un radical est dite 'substituée' lorsqu'elle comprend au moins un substituant choisi parmi les groupements :
   - hydroxyle,
   - alcoxy en C₁-C₄, (poly)hydroxyalcoxy en C₂-C₄,
   - amino, amino substitué par un ou plusieurs groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle à 5 ou 6 chaînons, comprenant éventuellement au moins un autre hétéroatome différent ou non de l'azote.
- Un radical aryle ou hétéroaryle ou la partie aryle ou hétéroaryle d'un radical est dite 'substituée' lorsqu'elle comprend au moins un substituant porté par un atome de carbone, choisi parmi
   - un radical alkyle en C₁-C₁₆, de préférence en C₁-C₈, éventuellement substitué par un ou plusieurs radicaux choisis parmi les radicaux hydroxy, alcoxy en C₁-C₂, (poly)-hydroxyalcoxy en C₂-C₄, , acylamino, amino substitué par deux radicaux alkyles, identiques ou différents, en C₁-C₄ , éventuellement porteurs d'au moins un groupement hydroxyle ou, les deux radicaux pouvant former avec l'atome d'azote auquel ils sont rattachés, un hétérocycle à 5 à 7 chaînons, de préférence de 5 ou 6 chaînons, comprenant éventuellement un autre hétéroatome identique ou différent de l'azote ;
   - un atome d'halogène tel que chlore, fluor ou brome ;
   - un groupement hydroxyle
   - un radical alcoxy en C₁-C₂ ; un radical (poly)-hydroxyalcoxy en C₂-C₄ ;
   - un radical amino ; un radical amino substitué par un ou deux radicaux alkyles, identiques ou différents, en C₁-C₄ éventuellement porteurs d'au moins un groupement hydroxyle ou amino par deux radicaux alkyles en C₁-C₂ éventuellement substitués ;
   - un radical acylamino (-NR-COR') dans lequel le radical R est un atome d'hydrogène, un radical alkyle en C₁-C₄ éventuellement porteur d'au moins un groupement hydroxyle et le radical R' est un radical alkyle en C₁-C₂ ; un radical carbamoyle ((R)₂N-CO-) dans lequel les radicaux R, identiques ou non, représentent un atome d'hydrogène, un radical alkyle en C₁-C₄ éventuellement porteur d'au moins un groupement hydroxyle ; un radical alkylsulfonylamino (R'SO₂-NR-) dans lequel le radical R représente un atome d'hydrogène, un radical alkyle en C₁-C₄ éventuellement porteur d'au moins un groupement hydroxyle et le radical R' représente un radical alkyle en C₁-C₄, un radical phényle ; un radical aminosulfonyle ((R)₂N-SO₂-) dans lequel les radicaux R, identiques ou non, représentent un atome d'hydrogène, un radical alkyle en C₁₋C₄ éventuellement porteur d'au moins un groupement hydroxyle.
- La Partie cyclique ou hétérocyclique d'un radical non aromatique est dite substituée lorsqu'elle comprend au moins un substituant porté par un atome de carbone choisi parmi les groupements :
   - hydroxyle,
   - alcoxy en C₁-C₄, (poly)hydroxyalcoxy en C₂-C₄,
   - alkylcarbonylamino ((RCO-NR'-) dans lequel le radical R' est un atome d'hydrogène, un radical alkyle en C₁-C₄ éventuellement porteur d'au moins un groupement hydroxyle et le radical R est un radical alkyle en C₁-C₂, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle à 5 ou 6 chaînons, comprenant éventuellement au moins un autre hétéroatome différent ou non de l'azote.

Comme indiqué auparavant, un premier objet de l'invention consiste en des composés correspondant à la formule (I) précitée.

Plus particulièrement, le composé de formule (I) Col1-LK-Col2, est tel que les radicaux R₁, R₂, R₃ et R'₁, indépendamment les uns des autres, représentent :
- un atome d'hydrogène ;
- un radical alkyle en C₁-C₆ éventuellement substitué
- un radical aryle, ou arylalkyle, tel que phényle, benzyle, la partie aryle étant éventuellement substituée ;
- les radicaux R₁, R₂, R₃ issus de W'₆ indépendamment les uns des autres peuvent éventuellement former avec une partie du groupement LK et avec l'atome d'azote ou d'oxygène auquel chacun est rattaché, un hétérocycle à 5 à 7 chaînons, saturé ou non, aromatique ou non éventuellement substitué ;
- le radical R'₁ issu de W₆ peut éventuellement former avec l'atome d'azote auquel il est rattaché et une partie du groupement LK, un hétérocycle comprenant de 5, 6 ou 7 chaînons, saturé ou non, aromatique ou non comprenant éventuellement un autre hétéroatome, choisi parmi l'azote, l'oxygène, et éventuellement substitué.

Conformément à un mode de réalisation préféré de l'invention, les radicaux R₁, R₂, R₃ et R'₁ identiques ou différents, représentent :
- un atome d'hydrogène ;
- un radical alkyle en C₁-C₃ éventuellement substitué, tel que méthyle, éthyle, 2-hydroxyéthyle, 2-méthoxyéthyle ;
- un radical phényle, éventuellement substitué par un ou plusieurs radicaux choisis parmi les radicaux hydroxyle, alcoxy en C₁-C₂, amino, amino substitués par un ou plusieurs groupements en C₁-C₄ éventuellement porteurs d'au moins un groupement hydroxyle ;
- le radical R'₁ issu de W₆ peut former avec l'atome d'azote, auquel il est rattaché et une partie du groupement LK, un hétérocycle à 5 ou 6 chaînons de type pyrrolidine, pipéridine, pipérazine, homopipérazine, éventuellement substitué par un ou plusieurs radicaux méthyle, hydroxy, amino, (di)méthylamino.

Selon une variante encore plus préférée de l'invention, les radicaux R₁, R₂, R₃ et R'₁, identiques ou différents, représentent :
- un atome d'hydrogène ;
- un radical méthyle, éthyle, 2-hydroxyéthyle ;
- un radical phényle éventuellement substitué par un radical hydroxy, méthoxy, amino, (di)méthylamino , (di)(2-hydroxyéthyl)amino ;
- le radical R'1 issu de W6 peut former avec l'atome d'azote auquel il est rattaché et une partie du groupement LK, un hétérocycle à 5 à 7 chaînons tels que la pyrrolidine, 3-hydroxypyrrolidine, 3-diméthylaminopyrrolidine, pipéridine, 2-(2-hydroxyéthyl pipéridine), 4-(aminométhyl)pipéridine, 4-(2-hydroxyéthyl)pipéridine, 4-(diméthyl amino)pipéridine, pipérazine, 1-méthylpipérazine, 1-(2-hydroxyéthyl)pipérazine, 1-(2-aminoéthyl)pipérazine, 1-hydroxyéthyléthoxypipérazine, homopipérazine, 1-méthyl-1,4-perhydrodiazépine.

Pour ce qui a trait aux radicaux R₄, R₅, R₆, R'₆, R₇, R₈, R₁₀, R₁₁, ces derniers, identiques ou différents, représentent plus particulièrement :
- un atome d'hydrogène pour R₄, R₅, R₇, R₈, R₁₀, R₁₁ ;
- un radical alkyle en C₁-C₁₆, de préférence en C₁-C₈, éventuellement substitué ;
- un atome d'halogène tel que chlore, fluor ou brome ;
- un groupement hydroxyle ;
- un radical alcoxy en C₁-C₂ ; un radical (poly)-hydroxyalcoxy en C₂-C₄ ;
- un radical amino ; un radical amino substitué par un ou deux radicaux alkyles, identiques ou différents, en C₁-C₄ éventuellement porteurs d'au moins un groupement hydroxyle ou dialkyle en C1-C4 amino ;
- un radical alkylcarbonylamino (RCO-NR'-) dans lequel le radical R représente un radical alkyle en C₁-C₄ et le radical R' représente un hydrogène, un radical alkyle en C₁-C₄ ; un radical carbamoyle ((R)₂N-CO-) dans lequel les radicaux R identiques ou non, représentent un atome d'hydrogène, un radical alkyle en C₁-C₄ éventuellement porteurs d'au moins un groupement hydroxyle ; un radical alkylsulfonylamino (R'SO₂-NR-) dans lequel le radical R représente un atome d'hydrogène, un radical alkyle en C₁-C₄ éventuellement porteur d'au moins un groupement hydroxyle, le radical R' représente un radical alkyle en C₁-C₄ ; un radical aminosulfonyle ((R)₂N-SO₂-) dans lequel les radicaux R identiques ou non, représentent un atome d'hydrogène, un radical alkyle en C₁-C₄ éventuellement porteur d'au moins un groupement hydroxyle ;
- une liaison de W'5 à W'6.

Plus préférentiellement, lesdits radicaux, identiques ou différents, représentent :
- un atome d'hydrogène pour R₄, R₅, R₆, R'₆, R₇, R₈, R₁₀, R₁₁ ;
- un radical alkyle en C₁-C₄ éventuellement substitué par un ou plusieurs radicaux choisis parmi les radicaux hydroxy, acylamino, amino substitué par deux radicaux alkyles, identiques ou différents, en C₁-C₂, éventuellement porteurs d'au moins un groupement hydroxyle, un radical alcoxy en C₁-C₂ ;
- un radical amino ; un radical amino substitué par un ou deux radicaux alkyles, identiques ou différents, en C₁-C₂, éventuellement porteurs d'au moins un groupement hydroxyle; un radical acylamino ; un radical carbamoyle ; un radical sulfonylamino ;
- un radical hydroxy ; un radical alcoxy en C₁-C₂ ;
- une liaison de W'₅ à W'₆.

Selon une variante préférée de l'invention, les radicaux R₄, R₅, R₆, R'₆, R₇, R₈, R₁₀, R₁₁, indépendamment les uns des autres, représentent :
- un atome d'hydrogène pour R₄, R₅, R₆, R'₆, R₇, R₈, R₁₀, R₁₁ ;
- un radical méthyle, éthyle, propyle, 2-hydroxyéthyle, méthoxy, éthoxy, 2-hydroxyéthyloxy, 3-hydroxypropyloxy, 2-méthoxyéthyle ;
- un radical sulfonylamino ; un radical amino, méthylamino, diméthylamino, 2-hydroxyéthylamino, 3-hydroxypropylamino, un radical acylamino ; un radical hydroxy ;
- un atome de chlore ;
- une liaison de W'₅ à W'₆.

En ce qui concerne le radical Rg, celui-ci représente un atome d'hydrogène, un radical alkyle en C₁-C₁₅ ; un radical monohydroxyalkyle en C₂-C₆ ; un radical polyhydroxyalkyle en C₂-C₆ ; un radical alcoxy(C₁-C₆)alkyle en C₂-C₆ ; un radical aryle éventuellement substitué, tel que phényle ; un radical arylalkyle éventuellement substitué, tel que benzyle ; un radical amidoalkyle en C₂-C₆ ; un radical aminoalkyle en C₂-C₆ dont l'amine est substituée par deux radicaux alkyle identiques ou différents en C₁-C₄ éventuellement substitué. De plus, le radical R9 est tel que l'atome directement relié à l'atome d'azote est un atome de carbone.

De préférence, R₉ représente un atome d'hydrogène, un radical alkyle en C₁-C₆, un radical monohydroxyalkyle en C₂-C₆ ; un radical polyhydroxyalkyle en C₂-C₆ ; un radical alcoxy(C₁-C₆)alkyle en C₂-C₆ ; un radical phényle éventuellement substitué par au moins un atome de chlore, un groupement hydroxyle, un groupement RCO-NH- dans lequel R représente un radical alkyle en C₁-C₄, un radical amino substitué par deux radicaux alkyle en C₁-C₄ identiques ou différents ; un radical benzyle ; un radical aminoalkyle en C₁-C₆ ; un radical aminoalkyle en C₁-C₆ dont l'amine est substituée par deux radicaux alkyle identiques ou différents en C₁-C₄. De plus, le radical R₉ est tel que l'atome directement relié à l'atome d'azote est un atome de carbone.

Selon une variante particulière de l'invention, W₂, W₅, W'₅, indépendamment l'un de l'autre, représentent un groupement de formule (a) ou (c).

Selon cette variante, X₁ représente de préférence un groupement CR₇

De préférence, selon cette variante X₂ représente ou un radical CR₈.

R₄, R₅, R₆, R'₆, R₇, R₈ indépendamment les uns des autres, ont les mêmes significations que précédemment.

En ce qui concerne les groupements W₃, W₄, ces derniers, pris indépendamment l'un de l'autre, représentent plus particulièrement un hétérocycle de formules (1) à (3) suivantes :

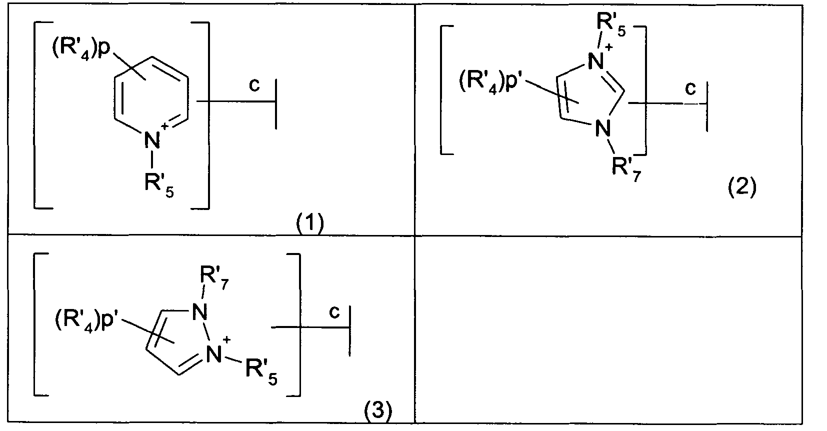

Dans lesquelles R'₄, R'₅, R'₇, p, p', a sont définis comme précédemment.

De préférence, R'₅ et R'₇ ont les mêmes définitions que Rg, à l'exception de l'hydrogène.

Conformément à un mode de réalisation préféré de l'invention, le groupement hétérocyclique aromatique cationique choisi parmi le 2-imidazolium, 2-benzimidazolium, 2-pyridinium, 3-pyridinium, 4-pyridinium, 2-quinolinium, 4-quinolinium, 3-pyrazolium, 4-pyrazolium, 3-indazolium, 4-indazolium, 5-indazolium, 6-indazolium, 7-indazolium.

Selon un mode de réalisation encore plus particulier de l'invention, les groupements W₃ et W₄ pris indépendamment l'un de l'autre, représente un hétérocycle aromatique cationique choisi parmi le 2-imidazolium, 2-pyridinium, 3-pyridinium, 4-pyridinium, 2-quinolinium, 4-quinolinium, 3-pyrazolium, 4-pyrazolium, 3-indazolium, 4-indazolium, 7-indazolium.

Dans le cas de W₃, les radicaux hétérocycliques cationiques sont rattachés au groupement LK par l'intermédiaire d'un atome d'azote quaternisé, c'est-à-dire par l'intermédiaire de R'₅.

Dans la formule (I), LK ne porte pas de charge cationique.

Plus précisément, LK représente une chaîne alkyle en C₂-C₂₀ linéaire ou ramifiée, ou cyclique, aromatique ou non :
- éventuellement interrompue par au moins un hétéroatome et/ou groupement comprenant au moins un hétéroatome, comme par exemple -O-, -NR'-, -CO-, SO₂-, à la condition qu'il n'y ait pas de groupe ou liaisons nitro, nitroso, peroxo dans le groupement LK ;
- éventuellement terminée par un hétéroatome ou groupement portant au moins un hétéroatome de préférence de préférence l'oxygène, l'azote, si LK est relié à W'5 ;
- éventuellement terminée par un groupement portant au moins un hétéroatome choisi parmi -CO- ou -SO₂- si LK est relié à W₆;
- si LK est relié à W₃ par l'intermédiaire d'un atome d'azote quaternisé, LK est directement relié à W₃ par un atome de carbone ; en d'autres termes LK ne peut donc être terminée par un hétéroatome ou un groupement comprenant au moins un hétéroatome ;
- éventuellement substituée par un ou plusieurs radicaux choisis parmi les radicaux hydroxy, alcoxy en C₁-C₂, (poly)-hydroxyalcoxy en C₂-C₄, amino substitué par un ou plusieurs groupements alkyle linéaires ou ramifiés en C₁-C₂ éventuellement porteurs d'au moins un groupement hydroxyle.

De préférence, LK représente une chaîne alkyle en C₂-C₂₀ linéaire ou ramifiée, éventuellement substituée par hydroxyle, amino substitué par plusieurs groupements alkyle linéaires ou ramifiés en C₁-C₄ éventuellement porteurs d'au moins un groupement hydroxyle. De plus, au cas où LK est relié à W'₅, LK peut être éventuellement terminé par au moins un hétéroatome ou groupement comprenant au moins un hétéroatome, comme par exemple -O-, -NR'-, -CO-, SO₂-.

Selon un autre mode de réalisation particulier de l'invention, LK représente une chaîne alkyle linéaire ou ramifiée en C₂-C₈, éventuellement substituée par un ou plusieurs radicaux choisis parmi les radicaux hydroxy, alcoxy en C₁-C₂, (di)alkylamino en C₁ -C₂, par un hétérocycle. De plus, au cas où LK est relié à W'₅, LK peut être éventuellement terminé par au moins un hétéroatome ou groupement comprenant au moins un hétéroatome, comme par exemple -O-, -NR'-, -CO-, SO₂-.

An est un anion ou un mélange d'anions organiques ou minéraux, afin de respecter l'électroneutralité du composé, par exemple choisis parmi les halogénures tels que chlorures, bromures, fluorures, iodures ; les hydroxyde ; les sulfates ; les hydrogénosulfates ; les alkyl(C₁-C₆)sulfates tels que par exemple le méthylsulfate ou l'éthylsulfate ; les phosphates ; les carbonates ; les hydrogénocarbonates ; les perchlorates ; les acétates ; les tartrates ; les citrates, les oxalates ; les alkyl(C₁-C₆)sulfonates tels que le méthylsulfonate ; les arylsulfonates substitués ou non par un radical alkyle en C₁-C₄ tels que par exemple un 4-toluylsulfonate.

Les sels d'addition avec un acide des composés de formule (I) peuvent être, à titre d'exemples, des halogénures, comme les chlorures, les bromures, des sulfates, des alkylsulfates pour lesquels la partie alkyle, linéaire ou ramifiée, est en C₁-C₆, comme les ions méthosulfates, éthosulfates, des hydrogénocarbonates, des perchlorates, des sels d'acides carboxyliques comme les acétates ; les citrates ; les tartrates, seuls ou combinés.

Conformément à un mode de réalisation préféré de l'invention, les composés correspondent notamment aux formules suivantes : 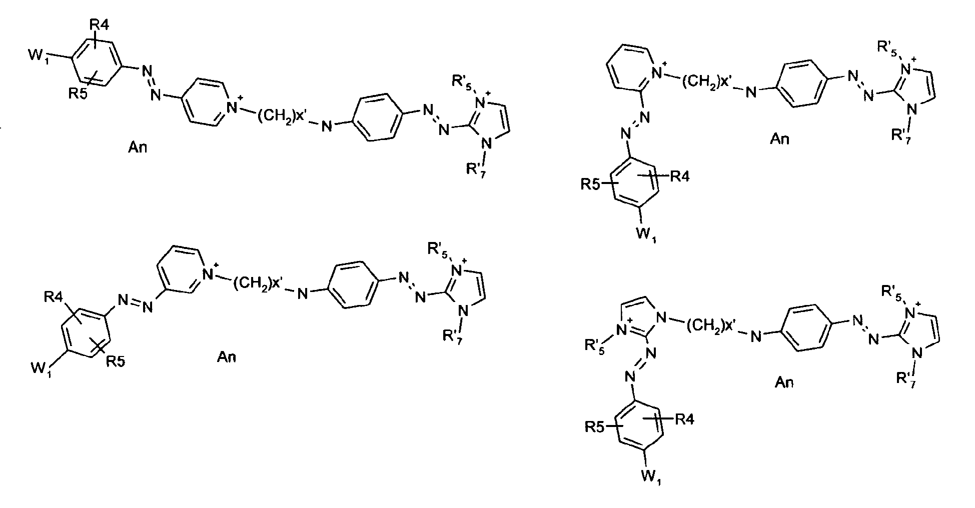 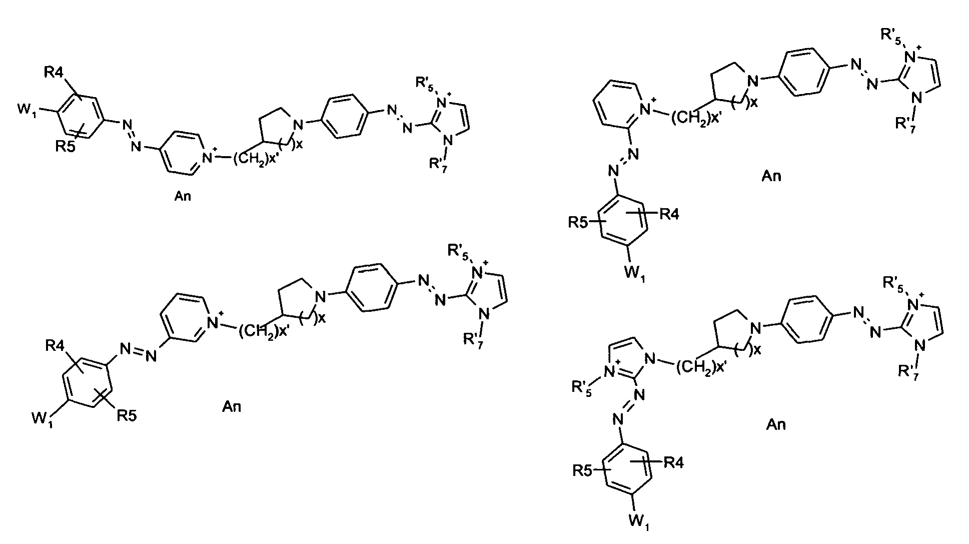 formules dans lesquelles W₁, R₄, R₅, R'₅, R'₇, sont tels que définis précédemment ;
- x, x', identiques ou différents, sont des nombres entiers compris entre 1 et 10, de préférence entre 1 et 6 ;
- l'électroneutralité de la molécule étant respectée par la présence d'un ou plusieurs anions An cosmétiquement acceptables et tels que définis précédemment.

Ces composés peuvent notamment être obtenus à partir des procédés de préparation décrits par exemple dans les documents, US 5708151, J.Chem. Res.., Synop. (1998), (10), 648-649, US3151106, US5852179, Hétérocycles, 1987, 26 (2) 313-317, Synth. Commun 1999, 29(13), 2271-2276, Tetrahedron, 1983, 39 (7), 1091-1101.

Un autre objet de la présente invention est constitué par une composition comprenant, dans un milieu approprié pour la teinture des fibres kératiniques, au moins un composé de formule (I) ou ses sels d'additions avec un acide, à titre de colorant direct.

La concentration en composé de formule (I) ou en chacun des composés de formule (I) peut varier entre 0,001 et 20 % en poids par rapport au poids total de la composition tinctoriale, plus particulièrement entre 0,01 à 10 % en poids, et de préférence entre 0,05 et 5 % en poids.

La composition selon l'invention peut de plus comprendre une base d'oxydation. Cette base d'oxydation peut être choisie parmi les bases d'oxydation classiquement utilisées en teinture d'oxydation, par exemple les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La composition selon l'invention peut contenir de plus un ou plusieurs coupleurs conventionnellement utilisés pour la teinture de fibres kératiniques. Parmi ces coupleurs, on peut notamment citer les métaphénylènediamines, les méta-aminophénols, les métadiphénols, les coupleurs naphtaléniques et les coupleurs hétérocycliques.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont en général présents en quantité comprise entre 0,001 et 10 % en poids du poids total de la composition tinctoriale et plus préférentiellement de 0,005 à 6 % en poids. La ou les bases d'oxydation sont présentes en quantité de préférence comprise entre 0,001 à 10 % en poids du poids total de la composition tinctoriale, et plus préférentiellement de 0,005 à 6 % en poids.

D'une manière générale, les sels d'addition avec un acide utilisables dans le cadre des compositions tinctoriales de l'invention pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

La composition selon l'invention peut éventuellement comprendre au moins un colorant direct additionnel différents des composés de formule (I). Celui-ci peut être choisi parmi les espèces cationiques ou non ioniques.

A titre d'exemples non limitatifs, on peut citer les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane et les colorants naturels, seuls ou en mélanges.

Il peut par exemple être choisi parmi les colorants benzéniques nitrés rouges ou orangés suivants :
- le 1-hydroxy-3-nitro-4-N-(y-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- le1-amino-3-nitro-6-hydroxybenzène.
- le 1-(β-aminoéthyl)amino-2-nitro-4-(β-hydroxyéthyloxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine,
- le 1-amino-2-nitro-4-hydroxy-5-méthylbenzène.

Le colorant direct additionnel peut aussi être choisi parmi les colorants directs benzéniques nitrés jaunes et jaune-verts, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyi)amino-3-nitro-méthyibenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.

On peut aussi mentionner les colorants directs benzéniques nitrés bleus ou violets, comme par exemple :
- le 1-(β-hydroxyéthyl)amino-4-N,N-bis-(β-hydroxyéthyl)amino 2-nitrobenzène,
- le 1-(y-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 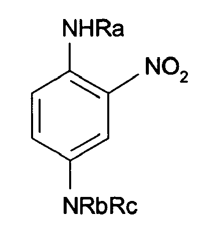
   dans laquelle :
   - Rb représente un radical alkyle en C₁-C₄, un radical β-hydroxyéthyle ou β-hydroxypropyle ou γ-hydroxypropyle ;
   - Ra et Rc, identiques ou différents, représentent un radical β-hydroxyéthyle, -β-hydroxypropyle, γ-hydroxypropyle, ou β,γ-dihydroxypropyle, l'un au moins des radicaux Rb, Rc ou Ra représentant un radical γ-hydroxypropyle et Rb et Rc ne pouvant désigner simultanément un radical β-hydroxyéthyle lorsque Rb est un radical γ-hydroxypropyle, telles que celles décrits dans le brevet français FR 2 692 572.

Parmi les colorants directs azoïques utilisables selon l'invention on peut citer les colorants azoïques cationiques décrits dans les demandes de brevets WO 95/15144, WO 95/01772 et EP 714954.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99

ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants figurant dans le tableau ci-dessous, An étant défini comme précédemment :

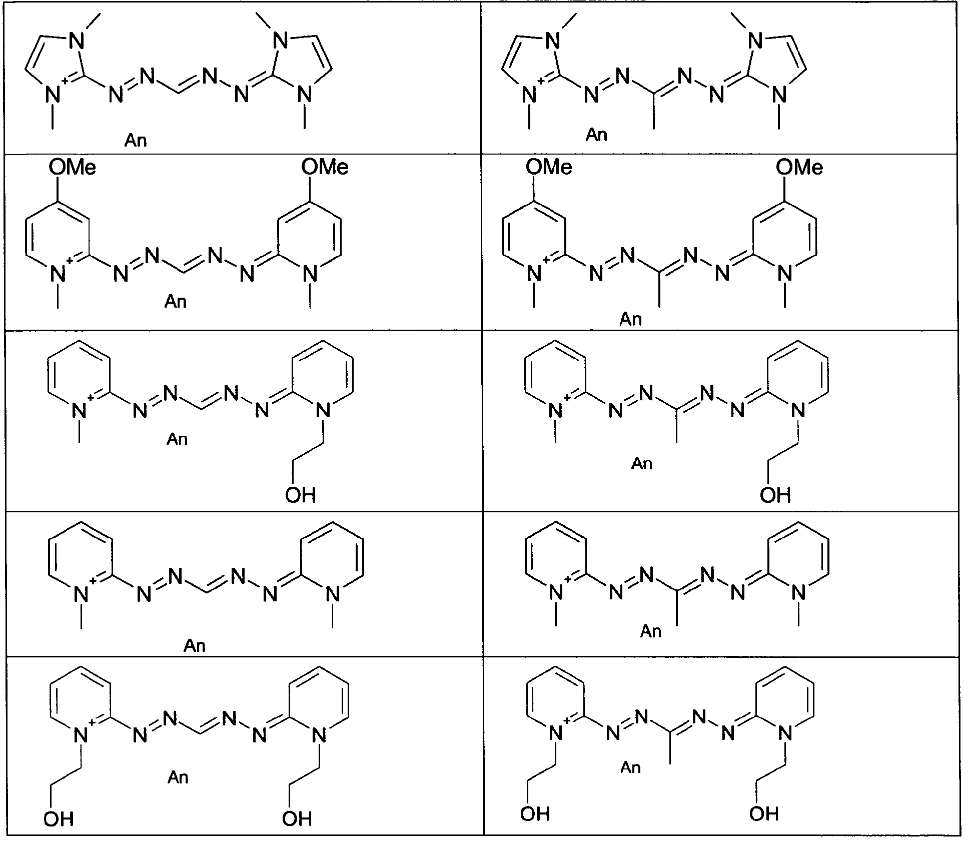

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

S'ils sont présents, la teneur en colorant(s) direct(s) additionnels dans la composition varie en général de 0,001 à 20% en poids par rapport au poids de la composition, et de préférence de 0,01 à 10% en poids par rapport au poids de la composition.

Le milieu approprié pour la teinture, appelé aussi support de teinture, est généralement constitué par de l'eau ou par un mélange d'eau et d'au moins un solvant organique pour solubiliser les composés qui ne seraient pas suffisamment solubles dans l'eau.

Plus particulièrement, les solvants organiques sont choisis parmi les monoalcools ou les diols, linéaires ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C₁-C₄, comme par exemple, le monoéthyléther ou le monobutyléther du diéthylèneglycol, seuls ou en mélange.

Les solvants usuels décrits ci-dessus, s'ils sont présents, représentent habituellement de 1 à 40 % en poids, plus préférentiellement de 5 à 30 % en poids, par rapport au poids total de la composition.

La composition tinctoriale conforme à l'invention peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 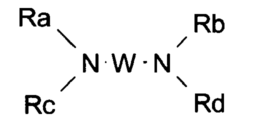
dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La composition selon l'invention peut en outre comprendre au moins un agent oxydant. On parle dans ce cas de composition prête à l'emploi.

Par composition prête à l'emploi, on entend au sens de la présente invention, la composition destinée à être appliquée immédiatement sur les fibres kératiniques, c'est à dire qu'elle peut être stockée telle quelle avant utilisation ou résulter du mélange extemporané de deux ou plusieurs compositions.

Ladite composition peut aussi être obtenue en mélangeant la composition selon l'invention avec une composition oxydante.

L'agent oxydant peut être n'importe quel agent oxydant utilisé de façon classique dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons telles que les uricases et les oxygénases à 4 électrons comme les laccases. L'utilisation du peroxyde d'hydrogène est particulièrement préférée.

La teneur en agent oxydant est en général comprise entre 1 et 40 % en poids, par rapport au poids de la composition prêt à l'emploi de préférence entre 1 et 20 % en poids par rapport au poids de la composition prêt à l'emploi.

Généralement, la composition oxydante utilisée est une composition aqueuse et peut se trouver sous la forme d'une solution ou encore d'une émulsion.

Habituellement, on mélange la composition exempte d'agent oxydant avec environ 0,5 à 10 équivalents en poids de la composition oxydante.

Notons que le pH de la composition prête à l'emploi est plus particulièrement compris entre 4 et 12, de préférence entre 7 et 11,5.

Le pH de la composition peut être réglé au moyen d'un agent alcalinisant ou acidifiant, notamment choisis parmi ceux mentionnés auparavant, dans le cadre de la description selon l'invention.

L'invention a aussi pour objet un procédé de coloration qui comprend l'application d'une composition tinctoriale selon l'invention sur les fibres kératiniques, sèches ou humides.

L'application sur les fibres de la composition tinctoriale comprenant le ou les composés de formule (I) ou ses sels d'addition avec un acide, éventuellement au moins une base d'oxydation éventuellement associée à au moins un coupleur, éventuellement au moins un colorant direct additionnel, peut être mise en oeuvre en présence d'agent oxydant.

Cet agent oxydant peut être ajouté à la composition comprenant le ou les composés de formule (I) ainsi que les éventuels base d'oxydation, coupleurs et/ou colorants directs additionnels, soit au moment de l'emploi, soit directement sur la fibre kératinique.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 4 et 12 environ, et encore plus préférentiellement entre 7 et 11,5. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Selon un mode de réalisation particulier, la composition selon l'invention est exempte de base d'oxydation et de coupleur.

La composition appliquée peut éventuellement comprendre au moins un agent oxydant.

La composition est donc appliquée sur les fibres kératiniques, sèches ou humides, puis laissée pendant un temps de pose suffisant pour obtenir la coloration recherchée.

Quelle que soit la variante retenue (avec ou sans agent oxydant) le temps de pose est en général compris entre quelques secondes et une heure, de préférence entre 3 et 30 minutes.

La température à laquelle la composition est laissée agir, est en général comprise entre 15 et 220°C, plus particulièrement entre 15 et 80°C, de préférence entre 15 et 40 °C.

A l'issue du temps de pose, la composition est éliminée par un rinçage à l'eau, suivi éventuellement d'un lavage avec un shampooing, puis éventuellement d'un séchage.

Un autre objet de l'invention est un dispositif à plusieurs compartiments ou "kit" de teinture dans lequel un premier compartiment renferme la composition tinctoriale de l'invention et un deuxième compartiment renferme la composition oxydante. Ce dispositif peut être équipé d'un moyen permettant de délivrer sur les cheveux le mélange souhaité, tel que les dispositifs décrits dans le brevet FR-2 586 913.

Les exemples qui suivent servent à illustrer l'invention sans toutefois présenter un caractère limitatif.

### EXEMPLES

### 1/ Synthèse des composés suivants :

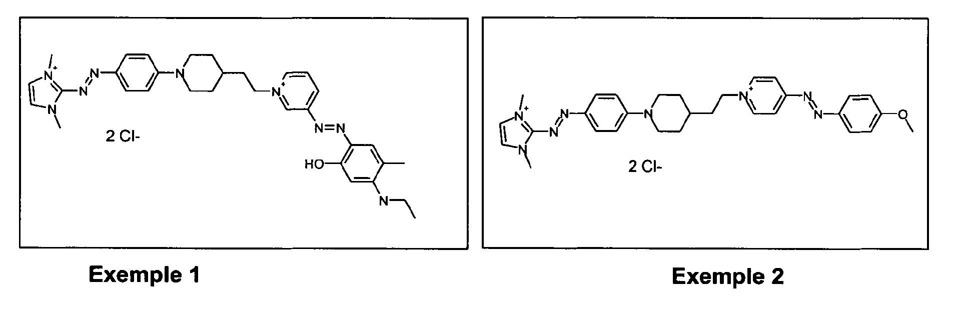

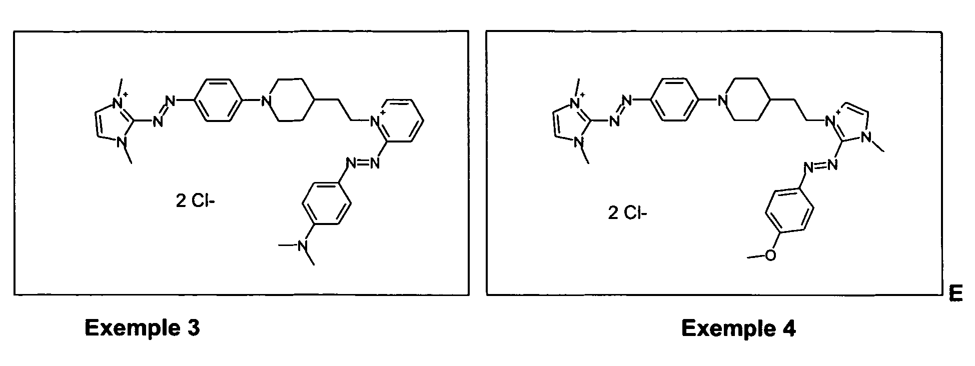

### Ces composés peuvent être obtenus de la façon suivante :

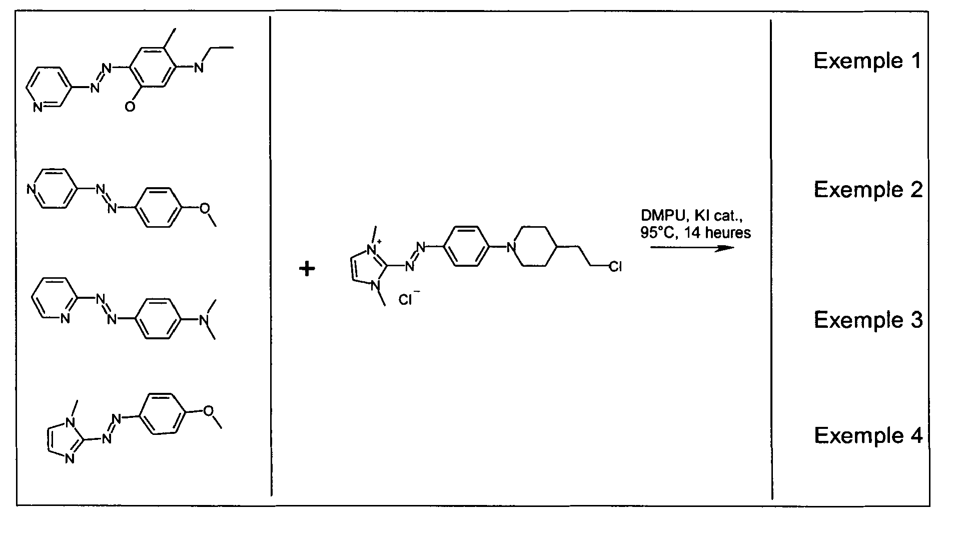

### Synthèse du dérivé halogéné :

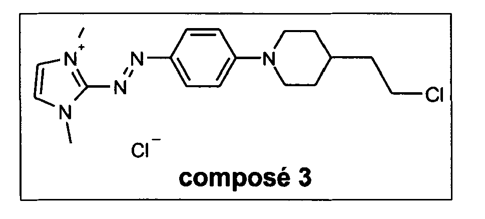

Le dérivé halogéné (composé 3) est préparé de la façon suivante : 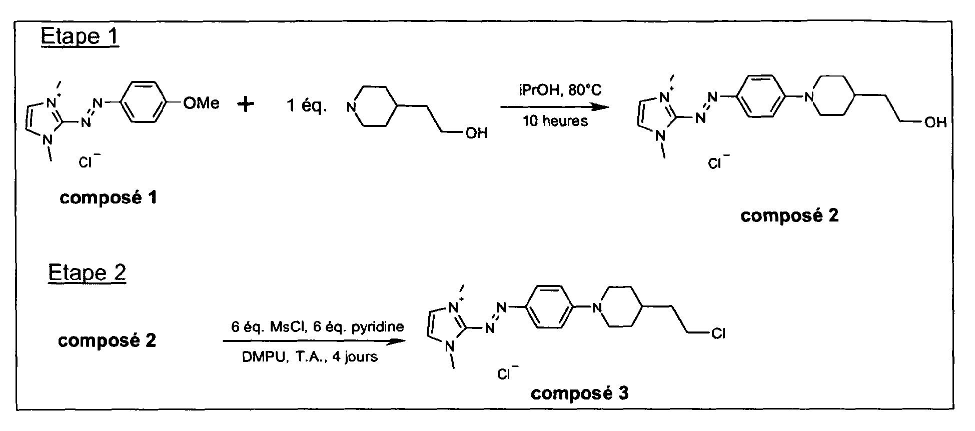
Le composé 1 est synthétisé suivant le brevet US5708151.

### Mode opératoire

### Etape 1

Dans un tricol, on place sous agitation le composé 1 (10g) dans 50 ml d'isopropanol ; l'amine (4.85 g ; 1 eq) préalablement solubilisé dans 20 ml d'isopropanol est alors rajouté au milieu réactionnel. On chauffe sous agitation à 80°C (température extérieure);

Après 10 heures, on arrête le chauffage et on laisse refroidir à température ambiante.

Après précipitation du produit dans un mélange acétone/acétate d'éthyle (100 ml/ 400 ml) puis filtration, on récupère 10.83 g d'un solide violet-rouge.

Les spectres RMN et masse sont en accord avec le composé 2.

### Etape 2

Dans un tricol, on place le composé 2 (1g), la 1,3-diméthyle-3,4,5,6-tétrahydro-2(1H)-pyrimidinone (20ml), le chlorure de méthanesulfonyle (1.27 ml, 6 eq) et la pyridine (1.3 g, 6 éq.).

On laisse sous agitation à T.A. pendant 4 jours.

Le produit est précipité dans de l'acétate d'éthyle puis filtré. Le précipité est solubilisé dans l'eau puis extrait au dichlorométhane. La phase organique est séchée sur sulfate de sodium, filtrée, puis concentrée.

On récupère le composé 3 sous forme d'une poudre rouge foncée mat.

Les spectres RMN et masse sont en accord avec le composé 3.

### Synthèse des exemples 1 à 4 :

Les 4 exemples ont été synthétisés suivant le même protocole opératoire :

Dans un ballon, on dispose le composé 3 (2,61 mmoles), le second colorant (2,61 mmoles), la 1,3-diméthyle-3,4,5,6-tétrahydro-2(1H)-pyrimidinone (4ml) et le KI en quantité catalytique (10 %) à 95°C pendant 14 heures. Les produits sont par la suite précipités dans de l'acétate d'éthyle puis purifiés par chromatographie.

### Synthèse de l'exemple 1 :

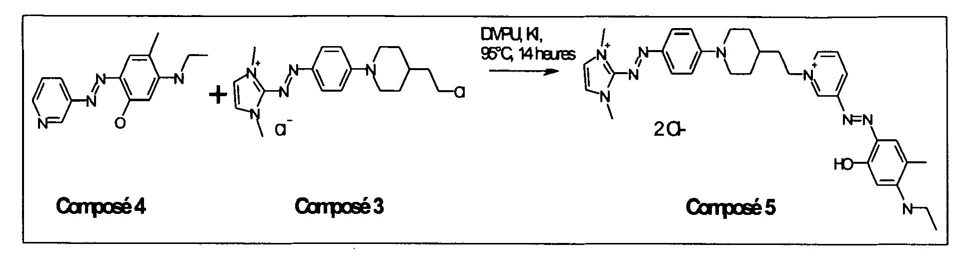

Le composé 4 est obtenu par réaction de couplage du sel de diazonium de la 3-aminopyridine sur la 3-(ethylamino)-4-méthylphénol en milieu acide.

### Quantités engagées :

Composé 4 : 67 mg
Composé 3 : 100 mg

Les analyses réalisées sont en accord avec la structure proposée pour le composé 5.

### Exemple de teinture :

On prépare la composition suivante :

| **Ingrédients** | **qté** |
|---|---|
| Alkyl (C8/C10 50/50) polyglucoside (2) en solution aqueuse à 60 % tamponnée | 12 g |
| Ethanol absolu pur | 20 g |
| Alcool benzylique pur | 4 g |
| Polyéthylène glycol(8 OE) 400 | 6 g |
| Eau déminéralisée | qsp 100 g |

On dissout 5.10⁻³ mol/l de composé 6 dans la composition ci-dessus décrite.

On applique la composition ainsi obtenue sur des mèches de cheveux à 90% de blancs.

On obtient avec le composé 5 une teinture rouge orangé.

### Synthèse de l'exemple 2 :

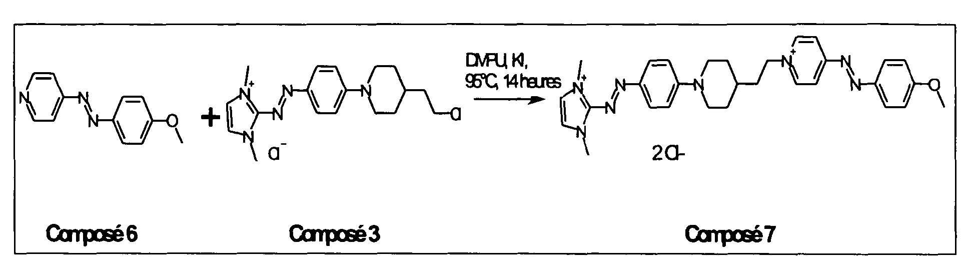

Le composé 6 est obtenu suivant le protocole opératoire décrit dans la publication Tetrahedron, 1983, 39 (7), 1091-1101.

### Quantités engagées :

Composé 6 : 55.8 mg
Composé 3 : 100 mg

Les analyses réalisées sont en accord avec la structure proposée pour le composé 7.

### Exemple de teinture :

On procède comme dans l'exemple 1 précédent.

On obtient avec le composé 7 une teinture rouge orangé.

### Synthèse de l'exemple 3 :

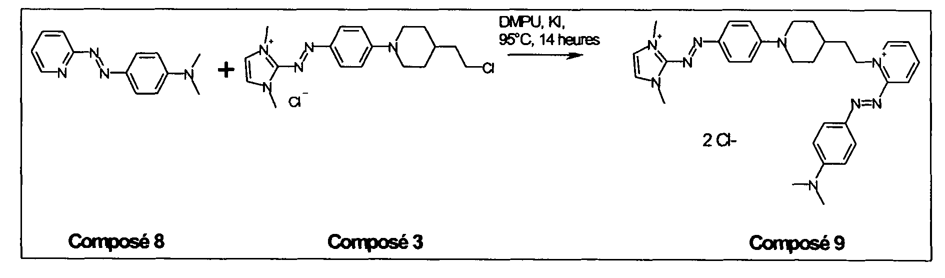

Le composé 8 est un produit disponible dans le commerce.

### Quantités engagées :

Composé 8 : 59.2 mg
Composé 3 : 100 mg

Les analyses réalisées sont en accord avec la structure proposée pour le composé 9.

### Exemple de teinture :

On procède comme dans l'exemple 1 précédent.

On obtient avec le composé 9 une teinture violette résistante.

### Synthèse de l'exemple 4 :

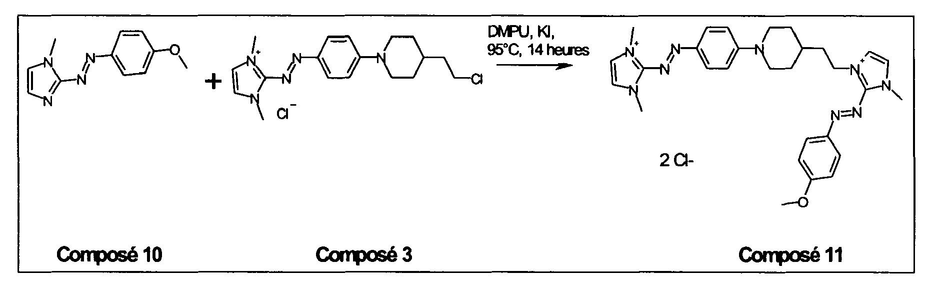

Le composé 10 est obtenu en 3 étapes :
- Réaction de couplage du sel de diazonium de la p-aminophénol sur l'imidazole.
- Réaction de O-alkylation en milieu acide à reflux dans le méthanol.
- Réaction de N-alkylation en présence de diméthylcarbonate et de K₂CO₃ à reflux.

### Quantités engagées :

Composé 10 : 56.6 mg
Composé 3 : 100 mg

Les analyses réalisées sont en accord avec la structure proposée pour le composé 11.

### Exemple de teinture :

On procède comme dans l'exemple 1 précédent.

On obtient avec le composé 11 une teinture rouge-fushia.

### 2/ Synthèse de l'exemple 5 :

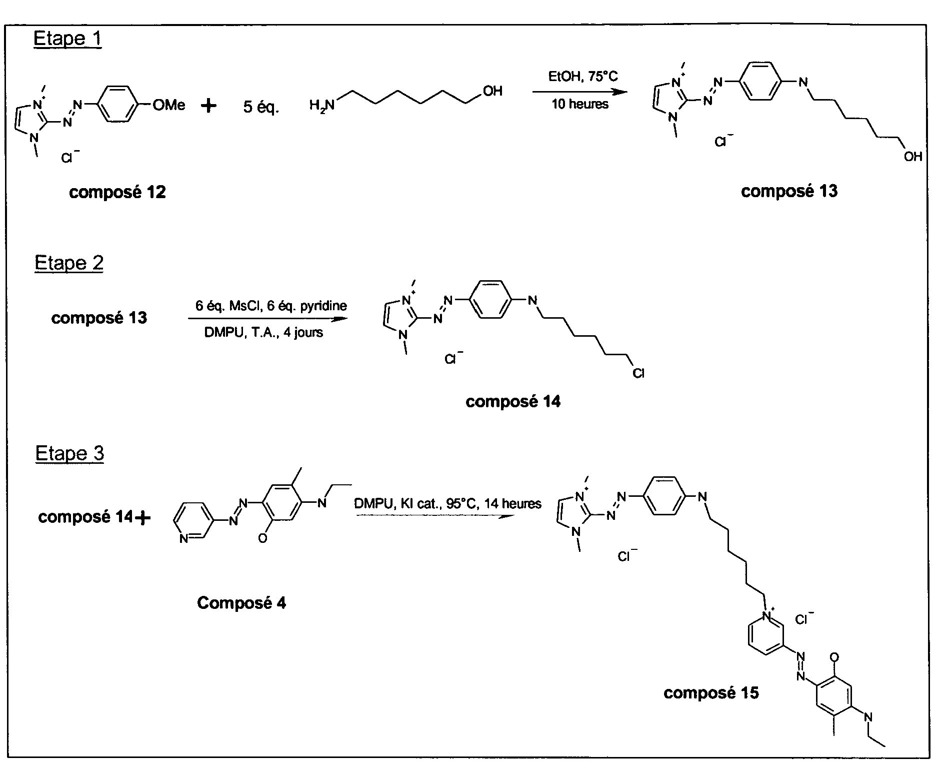

### Mode opératoire

### Etape 1

Dans un tricol, on place le composé 12 (20 g), l'éthanol (200ml) et on introduit la 6-aminohexanol (43.9 g, 5 eq).

On chauffe sous agitation à 70°C (température extérieure).

Après 10 heures, on arrête le chauffage et laisser refroidir à température ambiante.

Après évaporation du solvant, on récupère le composé 13.

On obtient un solide rouge. Les spectres RMN et masse sont en accord avec le composé 13.

### Etape 2

Dans un tricol, on place le composé 13 (3g), la 1,3-diméthyle-3,4,5,6-tétrahydro-2(1H)-pyrimidinone (80ml), le chlorure de méthanesulfonyle (4 ml, 6 eq) et la pyridine (4,12 ml, 6 éq.).

On laisse sous agitation à T.A. pendant 4 jours.

Le produit est précipité dans de l'acétate d'éthyle puis filtré. Le précipité est solubilisé dans l'eau puis extrait au dichlororométhane. La phase organique est séchée sur sulfate de sodium, filtrée, puis concentrée.

On récupère le composé 14 sous forme d'une poudre rouge mat.

Les spectres RMN et masse sont en accord avec le composé 14.

### Etape 3

Dans un tricol, on place les composés 14 (100 mg) et 4 (69 mg) , la 1,3-diméthyle-3,4,5,6-tétrahydro-2(1H)-pyrimidinone (4ml) et le KI en quantité catalytique,

On laisse sous agitation à 95°C. pendant 14 heures.

Le produit est précipité dans de l'acétate d'éthyle puis filtré. Le précipité est purifié par chromatographie.
Les analyses réalisées sont en accord avec la structure proposée pour le composé 15.

### Exemple de teinture :

On procède comme dans l'exemple 1 précédent.

On obtient avec le composé 15 une teinture rouge-orangée.

## Revendications

1. Composés cationiques de formule (I) suivante ou leurs sels d'addition avec un acide :
Col1 - LK - Col2 (I)
Dans laquelle Col1 et Col2 représentent : 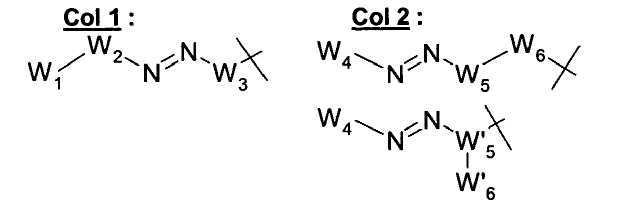 Dans lesquelles
**W**_{**1**} **et W'**_{**6**} indépendamment l'un de l'autre représentent un groupement -NR₁R₂, -OR3 dans lesquels R₁, R₂ et R₃ indépendamment l'un de l'autre représentent un atome d'hydrogène, une chaîne hydrocarbonée en C₁-C₂₀, de préférence en C₁-C₁₆, saturée ou insaturée, aromatique ou non, pouvant former un ou plusieurs cycles carbonés éventuellement substitués, comportant de 3 à 7 chaînons, éventuellement substituée, éventuellement interrompue par un ou plusieurs hétéroatomes ou par un ou plusieurs groupements portant au moins un hétéroatome, de préférence choisi parmi l'oxygène, l'azote ; R₁ et R₂ pouvant éventuellement former avec l'atome d'azote auquel ils sont rattachés, un hétérocycle à 5 à 7 chaînons, éventuellement substitué et comprenant éventuellement un autre hétéroatome identique ou différent de l'azote ;
Les radicaux R₁ , R₂, R₃ issus de W'₆ indépendamment les uns des autres peuvent éventuellement former avec une partie du groupement LK et avec l'atome d'azote ou d'oxygène auquel chacun est rattaché, un hétérocycle à 5 ou 7 chaînons, saturé ou non, aromatique ou non éventuellement substitué ;
**W**₆, représente un groupement -NR'₁-, -O- dans lequel R'₁, représente un atome d'hydrogène, une chaîne hydrocarbonée en C₁-C₂₀, de préférence en C₁-C₁₆, saturée ou insaturée, aromatique ou non, pouvant former un ou plusieurs cycles carbonés éventuellement substitués, comportant de 3 à 7 chaînons, éventuellement substituée, éventuellement interrompue par un ou plusieurs hétéroatomes ou par un ou plusieurs groupements portant au moins un hétéroatome, de préférence choisi parmi l'oxygène, l'azote ;
Le radical R'₁ issu de W₆ peut éventuellement former avec l'atome d'azote auquel il est rattaché et une partie du groupement LK, un hétérocycle comprenant de 5 à 7 chaînons, saturé ou non, aromatique ou non comprenant éventuellement un autre hétéroatome, choisi parmi l'azote, l'oxygène, et pouvant être éventuellement substitué ;
**W**_{**2**}**, W**_{**5**} et **W'**_{**5**}**,** indépendamment les uns des autres, représentent un groupement de formules (a) à (c) suivantes : 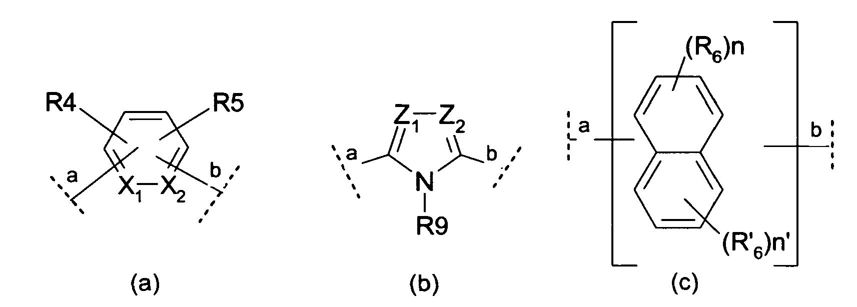
Formules dans lesquelles :
- X₁ représente un atome d'azote ou un groupement CR₇ ;
- X₂ représente un atome d'azote ou un groupement CR₈ ;
- Z₁ représente un atome d'azote ou un groupement CR₁₀ ;
- Z₂ représente un atome d'azote ou un groupement CR₁₁ ;
- R₄, R₅, R₆, R'₆, R₇, R₈, R₁₀, R₁₁, indépendamment les uns des autres, représentent :
- une chaîne hydrocarbonée en C₁-C₁₆, linéaire ou ramifiée, saturée ou insaturée, aromatique ou non, pouvant former un ou plusieurs cycles carbonés comportant de 3 à 6 chaînons, éventuellement substituée, éventuellement interrompue par un ou plusieurs hétéroatomes ou par un ou plusieurs groupements portant au moins un hétéroatome, de préférence choisi parmi l'oxygène, l'azote ;
- un groupement hydroxyle,
- un groupement alcoxy en C₁-C₄, un groupement (poly)hydroxyalcoxy en C₂-C₄ ; un groupement alcoxycarbonyle (RO-CO-) dans lequel R représente un radical alkyle en C₁-C₄, un radical alkylcarbonyloxy (RCO-O-) dans lequel R représente un radical alkyle est en C₁-C₄ ;
- un groupement amino, un groupement amino substitué par un ou plusieurs radicaux alkyles en C₁-C₄, identiques ou différents, éventuellement porteurs d'au moins un groupement hydroxyle, les deux radicaux alkyle pouvant éventuellement former avec l'atome d'azote auquel ils sont rattachés, un hétérocycle à 5 ou 6 chaînons éventuellement porteur d'un autre hétéroatome identique ou différent de l'azote ; un groupement alkylcarbonylamino (RCO-NR-) dans lequel les radicaux R indépendamment les uns des autres représentent un radical alkyle en C₁-C₄ ; un groupement carbamoyle ((R)₂N-CO) dans lequel les radicaux R indépendamment les uns des autres , identiques ou non, représentent un atome d'hydrogène, un radical alkyle en C₁-C₄ ; un groupement uréido (N(R)₂-CO-NR'-) dans lequel les radicaux R et R' , indépendamment les uns des autres, représentent un atome d'hydrogène, un radical alkyle en C₁-C₄ ; un groupement sulfonamide ((R)₂N-SO₂-) dans lequel les radicaux R, indépendamment les uns des autres, représentent un atome d'hydrogène, un radical alkyle en C₁-C₄, ; un groupement alkylsulfonylamino (RSO₂-NR'-) dans lequel les radicaux R, R', indépendamment les uns des autres, représentent un atome d'hydrogène, un radical alkyle en C₁-C₄ ; un groupement guanidinium ((R')₂N-C(=NH₂⁺)-NR-) dans lequel les radicaux R et R', indépendamment les uns des autres, représentent un atome d'hydrogène, un radical alkyle en C₁-C₄ ;
• un groupement nitro ; un groupement cyano ; un atome d'halogène, de préférence le chlore, le fluor ;
• R₄, R₅, R₆, R'₆, R₇, R₈, R₁₀, R₁₁ peuvent représenter un atome d'hydrogène ;
• R₄, R₅, R₆, R'₆, R₇, R₈, R₁₀, R₁₁, indépendamment les uns des autres, peuvent éventuellement former avec tout ou partie des groupements W'₁ ou W'₆ un hétérocycle comprenant de 5 à 7 chaînons, saturé ou non, éventuellement substitué ;
- une liaison de W'5 à W'6, ou au groupement LK ;
- a représente la liaison de W₂, W₅, W'₅ au groupement azoïque -N=N- ;
- b représente la liaison de W₂ à W₁, de W₅ à W₆, de W'₅ à W'₆;
- R₉ représente :
- un atome d'hydrogène,
- une chaîne hydrocarbonée en C₁-C₁₆, linéaire ou ramifiée, saturée ou insaturée, aromatique ou non, pouvant former un ou plusieurs cycles carbonés comportant de 3 à 7 chaînons, éventuellement substituée,
- n et n' représentent des nombres entiers et leur somme est inférieure ou égale à 6 ; il est à noter que lorsque la somme de n et n' est inférieure à 6, chaque substituant maquant est un atome d'hydrogène ;
**W**_{**3**}**, W**_{**4**}**,** représentent, indépendamment l'un de l'autre, un radical hétéroaromatique cationique représentés par l'une des formules (1) et (11) suivantes : 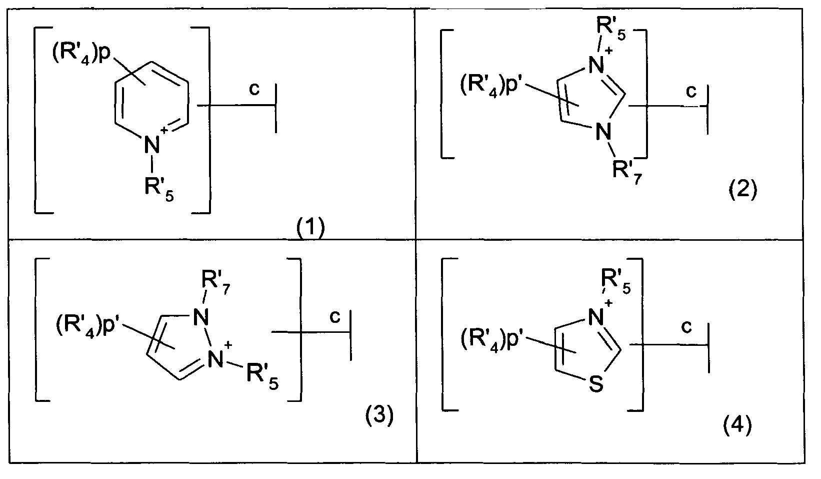 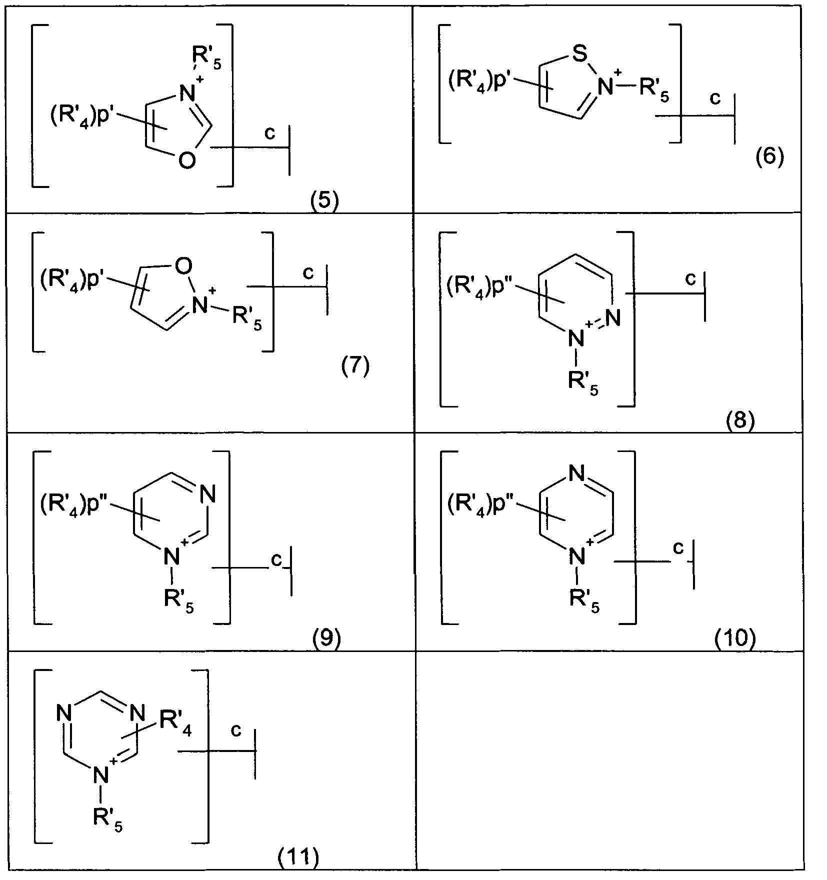
Dans lesquelles :
- R'₄ identiques ou différents, substituant le cycle principal, représentent :
• une chaîne hydrocarbonée en C₁-C₁₆, linéaire ou ramifiée, saturée ou insaturée, aromatique ou non, pouvant former un ou plusieurs cycles carbonés comportant de 3 à 6 chaînons, éventuellement substituée, éventuellement interrompue par un ou plusieurs hétéroatomes ou par un ou plusieurs groupements portant au moins un hétéroatome, de préférence choisi parmi l'oxygène, l'azote ;
• un groupement hydroxyle,
• un groupement alcoxy en C₁-C₄, un groupement (poly)hydroxyalcoxy en C₂-C₄ ; un groupement alcoxycarbonyle (RO-CO-) dans lequel R représente un radical alkyle en C₁-C₄, un radical alkylcarbonyloxy (RCO-O-) dans lequel R représente un radical alkyle est en C₁-C₄ ;
• un groupement amino, un groupement amino substitué par un ou plusieurs radicaux alkyles en C₁-C₄, indépendamment les uns des autres, éventuellement porteurs d'au moins un groupement hydroxyle, les deux radicaux alkyle pouvant éventuellement former avec l'atome d'azote auquel ils sont rattachés, un hétérocycle à 5 ou 6 chaînons éventuellement porteur d'un autre hétéroatome identique ou différent de l'azote ; un groupement alkylcarbonylamino (RCO-NR'-) dans lequel le radical R représente un radical alkyle en C₁-C₄ et le radical R' représente un hydrogène, un radical alkyle en C₁-C₄ ; un groupement carbamoyle ((R)₂N-CO-) dans lequel les radicaux R indépendamment les uns des autres, représentent un atome d'hydrogène, un radical alkyle en C₁-C₄ ; un groupement uréido (N(R)₂-CO-NR'-) dans lequel les radicaux R et R', indépendamment les uns des autres, représentent un atome d'hydrogène, un radical alkyle en C₁-C₄ ; un groupement sulfonamide ((R)₂N-SO₂-) dans lequel les radicaux R indépendamment les uns des autres, représentent un atome d'hydrogène, un radical alkyle en C₁-C₄ ; un groupement alkylsulfonylamino (RSO₂-NR'-) dans lequel les radicaux R, R', indépendamment les uns des autres, représentent un atome d'hydrogène, un radical alkyle en C₁-C₄ ; un groupement guanidinium ((R')₂N-C(=NH₂⁺)-NR-) dans lequel les radicaux R et R', indépendamment les uns des autres, représentent un atome d'hydrogène, un radical alkyle en C₁-C₄ ;
• un groupement nitro ; un groupement cyano ; un atome d'halogène, de préférence le chlore, le fluor ;
• deux radicaux R'₄ portés par deux atomes de carbone adjacents du cycle principal peuvent éventuellement former un cycle secondaire, aromatique ou non, à 5 ou 6 chaînons, éventuellement substitué par au moins un hydrogène ; au moins un groupement hydroxyle ; au moins un radical alkyle en C₁-C₄ ; au moins un radical alcoxy en C₁-C₄ ; au moins un radical (poly)hydroxyalcoxy en C₂-C₄ ; au moins un radical amino ; au moins un radical amino substitué par un ou plusieurs radicaux identiques ou différents, alkyle en C₁-C₄, éventuellement porteurs d'au moins un groupement hydroxyle ; de préférence, le cycle secondaire est un cycle aromatique à 6 chaînons éventuellement substitué comme indiqué précédemment ;
- R'₅ porté par l'atome d'azote quaternisé, dans le cas de W₄, représente une chaîne hydrocarbonée en C₁-C₁₆, linéaire ou ramifiée, saturée ou insaturée, aromatique ou non, pouvant former un ou plusieurs cycles carbonés éventuellement substitués comportant de 3 à 6 chaînons, éventuellement substituée, éventuellement interrompue par un ou plusieurs hétéroatomes ou par un ou plusieurs groupements portant au moins un hétéroatome, de préférence choisi parmi l'oxygène, l'azote ; le radical R'₅ est tel que l'atome directement relié à l'atome d'azote quaternisé est un atome de carbone ;
- R'₅ porté par l'atome d'azote quaternisé, dans le cas de W₃, représente une liaison à LK ;
- R'₇ représente un radical alkyle en C₁-C₄ éventuellement substitué ; un radical phényle éventuellement substitué ; un radical benzyle éventuellement substitué ;
- La liaison c relie le radical cationique défini par les formules (1) à (11) au groupement azoïque ; ladite liaison pouvant se trouver sur le cycle principal ou secondaire ; de préférence, la liaison a avec le groupement azoïque se trouve sur le cycle principal ;
- p est un nombre entier variant de 0 à 4, p' est un nombre entier variant de 0 à 2 et p" est un nombre entier variant de 0 à 3 ; il est à noter que lorsque le cycle principal ne porte pas le nombre de substituant maximum, alors la position non substituée porte un atome d'hydrogène ;
**LK** représente une chaîne hydrocarbonée en C₂-C₄₀, de préférence en C₂-C₂₀, saturée ou non, linéaire ou ramifiée, cyclique ou non, aromatique ou non, éventuellement substituée, éventuellement interrompue par au moins un hétéroatome ou groupement comprenant au moins un hétéroatome, de préférence l'oxygène, l'azote ; le groupement LK ne comporte par ailleurs pas de liaison ou groupement peroxo, nitro, nitroso ; si LK est relié à W'₅, LK peut être terminé par un hétéroatome ou groupement comprenant au moins un hétéroatome, de préférence l'oxygène, l'azote ; si LK est relié à W₆, LK peut être terminé par un groupement comprenant au moins un hétéroatome choisi parmi -CO-, -SO₂- ; si LK est relié à W₃, la liaison se fait par l'intermédiaire d'un atome de carbone ;
L'électroneutralité des composés étant assurée par un ou plusieurs anions An cosmétiquement acceptables.

2. Composés selon la revendication précédente, **caractérisés en ce que** R₁, R₂, R₃ et R'₁, indépendamment les uns des autres, représentent :
- un atome d'hydrogène ;
- un radical alkyle en C1-C6 éventuellement substitué
- un radical aryle, ou arylalkyle, tel que phényle, benzyle, la partie aryle étant éventuellement substituée ;
- les radicaux R₁, R₂, R₃ issus de W'₆ indépendamment les uns des autres peuvent éventuellement former avec une partie du groupement LK et avec l'atome d'azote ou d'oxygène auquel chacun est rattaché, un hétérocycle à 5 à 7 chaînons, saturé ou non, aromatique ou non éventuellement substitué ;
- le radical R'₁ issu de W₆ peut éventuellement former avec l'atome d'azote auquel il est rattaché et une partie du groupement LK, un hétérocycle comprenant de 5, 6 ou 7 chaînons, saturé ou non, aromatique ou non comprenant éventuellement un autre hétéroatome, choisi parmi l'azote, l'oxygène, et éventuellement substitué.

3. Composés selon l'une quelconque des revendications précédentes, **caractérisés en ce que** les radicaux R₁, R₂, R₃ et R'₁ identiques ou différents, représentent :
- un atome d'hydrogène ;
- un radical alkyle en C₁-C₃ éventuellement substitué, tel que méthyle, éthyle, 2-hydroxyéthyle, 2-méthoxyéthyle ;
- un radical phényle, éventuellement substitué par un ou plusieurs radicaux choisis parmi les radicaux hydroxyle, alcoxy en C₁-C₂, amino, amino substitués par un ou plusieurs groupements en C₁-C₄ éventuellement porteurs d'au moins un groupement hydroxyle ;
- le radical R'₁ issu de W₆ peut former avec l'atome d'azote, auquel il est rattaché et une partie du groupement LK, un hétérocycle à 5 ou 6 chaînons de type pyrrolidine, pipéridine, pipérazine, homopipérazine, éventuellement substitué par un ou plusieurs radicaux méthyle, hydroxy, amino, (di)méthylamino.

4. Composés selon l'une des revendications précédentes, **caractérisés en ce que** les radicaux R₁, R₂, R₃ et R'₁, identiques ou différents, représentent :
- un atome d'hydrogène ;
- un radical méthyle, éthyle, 2-hydroxyéthyle ;
- un radical phényle éventuellement substitué par un radical hydroxy, méthoxy, amino, (di)méthylamino , (di)(2-hydroxyéthyl)amino ;
- le radical R'₁ issu de W₆ peut former avec l'atome d'azote auquel il est rattaché et une partie du groupement LK, un hétérocycle à 5 à 7 chaînons tels que la pyrrolidine, 3-hydroxypyrrolidine, 3-diméthylaminopyrrolidine, pipéridine, 2-(2-hydroxyéthyl pipéridine), 4-(aminométhyl)pipéridine, 4-(2-hydroxyéthyl)pipéridine, 4-(diméthyl amino)pipéridine, pipérazine, 1-méthylpipérazine, 1-(2-hydroxyéthyl)pipérazine, 1-(2-aminoéthyl)pipérazine, 1-hydroxyéthyléthoxy pipérazine, homopipérazine, 1-méthyl-1,4-perhydrodiazépine.

5. Composés selon l'une des revendications précédentes, **caractérisés en ce que** les radicaux R₄, R₅, R₆, R'₆, R₇, R₈, R₉, R₁₀, R₁₁, identiques ou différents, représentent :
- un atome d'hydrogène pour R₄, R₅, R₇, R₈, Rio, R₁₁ ;
- un radical alkyle en C₁-C₁₆, de préférence en C₁-C₈, éventuellement substitué ;
- un atome d'halogène tel que chlore, fluor ou brome ;
- un groupement hydroxyle ;
- un radical alcoxy en C₁-C₂ ; un radical (poly)-hydroxyalcoxy en C₂-C₄ ;
- un radical amino ; un radical amino substitué par un ou deux radicaux alkyles, identiques ou différents, en C₁-C₄ éventuellement porteurs d'au moins un groupement hydroxyle ou dialkyle en C₁-C₄ amino ;
- un radical alkylcarbonylamino (RCO-NR'-) dans lequel le radical R représente un radical alkyle en C₁-C₄ et le radical R' représente un hydrogène, un radical alkyle en C₁-C₄ ; un radical carbamoyle ((R)₂N-CO-) dans lequel les radicaux R identiques ou non, représentent un atome d'hydrogène, un radical alkyle en C₁-C₄ éventuellement porteurs d'au moins un groupement hydroxyle ; un radical alkylsulfonylamino (R'SO₂-NR-) dans lequel le radical R représente un atome d'hydrogène, un radical alkyle en C₁-C₄ éventuellement porteur d'au moins un groupement hydroxyle, le radical R' représente un radical alkyle en C₁-C₄ ; un radical aminosulfonyle ((R)₂N-SO₂-) dans lequel les radicaux R identiques ou non, représentent un atome d'hydrogène, un radical alkyle en C₁-C₄ éventuellement porteur d'au moins un groupement hydroxyle ;
- une liaison de W'₅ à W'₆.

6. Composés selon l'une des revendications précédentes, **caractérisés en ce que** les radicaux R₄, R₅, R₆, R'₆, R₇, R₈, R₉, R₁₀, R₁₁, identiques ou différents, représentent :
- un atome d'hydrogène pour R₄, R₅, R₆, R'₆, R₇, R₈, R₁₀, R₁₁ ;
- un radical alkyle en C₁-C₄ éventuellement substitué par un ou plusieurs radicaux choisis parmi les radicaux hydroxy, acylamino, amino substitué par deux radicaux alkyles, identiques ou différents, en C₁-C₂, éventuellement porteurs d'au moins un groupement hydroxyle, un radical alcoxy en C₁-C₂ ;
- un radical amino ; un radical amino substitué par un ou deux radicaux alkyles, identiques ou différents, en C₁-C₂, éventuellement porteurs d'au moins un groupement hydroxyle; un radical acylamino ; un radical carbamoyle ; un radical sulfonylamino ;
- un radical hydroxy ; un radical alcoxy en C₁-C₂ ;
- une liaison de W'₅ à W'₆.

7. Composés selon l'une des revendications précédentes, **caractérisés en ce que** W₂, W₅, W'5, identiques ou différents, représentent un composé de formule (a) ou (c).

8. Composés selon l'une des revendications précédentes, **caractérisés en ce que** X₁ représente un groupement CR₇.

9. Composés selon la revendication précédente, **caractérisés en ce que** X₂ représente un groupement CR₈.

10. Composés selon l'une des revendications précédentes, **caractérisés en ce que** W₃, W₄, pris indépendamment l'un de l'autre, représentent un hétérocycle de formules (1) à (3) suivantes : 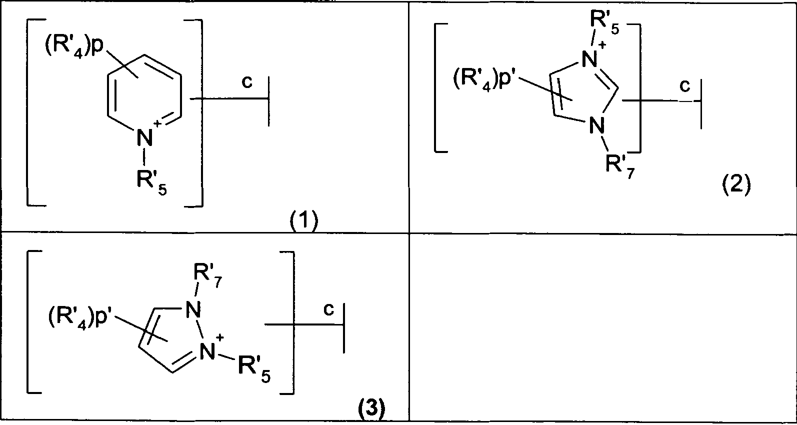
Dans lesquelles R'₄, R'₅, R'₇, p, p', a sont définis comme précédemment.

11. Composés selon la revendication précédente, **caractérisés en ce que** l'hétérocycle aromatique est choisi parmi le 2-imidazolium, 2-benzimidazolium, 2-pyridinium, 3-pyridinium, 4-pyridinium, 2-quinolinium, 4-quinolinium, 3-pyrazolium, 4-pyrazolium, 3-indazolium, 4-indazolium, 5-indazolium, 6-indazolium, 7-indazolium ; l'un au moins des deux groupements W₃ ou W₄ ne représente pas un groupement imidazolium non substitué.

12. Composés selon l'une des revendications précédentes, **caractérisés en ce que** LK ne porte pas de charge cationique.

13. Composés selon l'une des revendications précédentes, **caractérisés en ce que** LK représente une chaîne alkyle en C₁-C₂₀ linéaire ou ramifiée, ou cyclique, aromatique ou non :
- éventuellement interrompue par au moins un hétéroatome et/ou groupement comprenant au moins un hétéroatome, comme par exemple -O-, -NR'-, -CO-, SO₂-, à la condition qu'il n'y ait pas de groupe ou liaisons nitro, nitroso, peroxo dans le groupement LK ;
- éventuellement terminée par un hétéroatome ou groupement portant au moins un hétéroatome de préférence de préférence l'oxygène, l'azote, si LK est relié à W'₅ ;
- éventuellement terminée par un groupement portant au moins un hétéroatome choisi parmi -CO- ou -SO₂- si LK est relié à W₆;
- si LK est relié à W₃ par l'intermédiaire d'un atome d'azote quaternisé, LK est directement relié à W₃ par un atome de carbone ; en d'autres termes LK ne peut donc être terminée par un hétéroatome ou un groupement comprenant au moins un hétéroatome ;
- éventuellement substituée par un ou plusieurs radicaux choisis parmi les radicaux hydroxy, alcoxy en C₁-C₂, (poly)-hydroxyalcoxy en C₂-C₄, amino substitué par un ou plusieurs groupements alkyle linéaires ou ramifiés en C₁-C₂ éventuellement porteurs d'au moins un groupement hydroxyle.

14. Composés selon l'une quelconque des revendications précédentes **caractérisés en ce qu'**ils correspondent à la formule suivante : 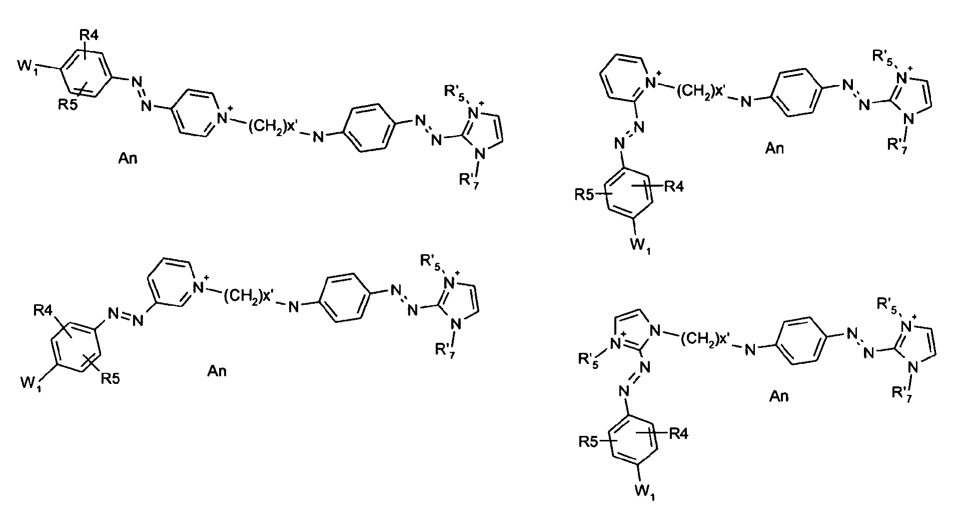 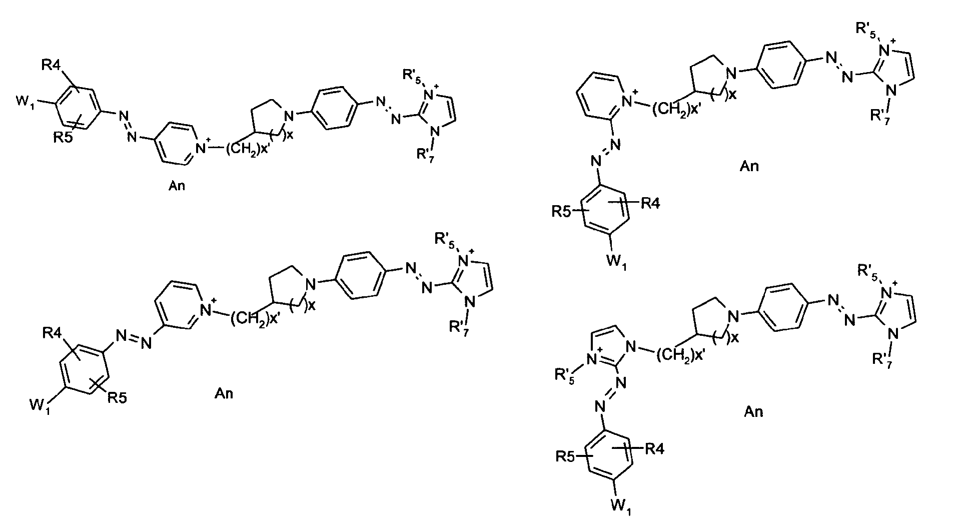 formules dans lesquelles W₁, R₄, R₅, R'₅, R'₇, sont tels que définis précédemment ;
- x, x', identiques ou différents, sont des nombres entiers compris entre 1 et 10, de préférence entre 1 et 6 ;
- l'électroneutralité de la molécule étant respectée par la présence d'un ou plusieurs anions An cosmétiquement acceptables et tels que définis précédemment.

15. Composés selon l'une quelconque des revendications précédentes, **caractérisés en ce que** l'anion cosmétiquement acceptable est choisi parmi les halogénures tels que chlorures, bromures, fluorures, iodures ; les hydroxyde ; les sulfates ; les hydrogénosulfates ; les alkyl(C₁-C₆)sulfates tels que par exemple le méthylsulfate ou l'éthylsulfate ; les phosphates ; les carbonates ; les hydrogénocarbonates ; les perchlorates ; les acétates ; les tartrates ; les citrates, les oxalates ; les alkyl(C₁₋C₆)sulfonates tels que le méthylsulfonate ; les arylsulfonates substitués ou non par un radical alkyle en C₁-C₄ tels que par exemple un 4-toluylsulfonate.

16. Composition tinctoriale comprenant dans un milieu approprié pour la teinture des fibres kératiniques, à titre de colorant direct, au moins un composé de formule (I), ou ses sels d'addition avec un acide, selon l'une quelconque des revendications précédentes.

17. Composition selon la revendication précédente, **caractérisée en ce que** la teneur en composé de formule (I) ou en chacun des composés de formule (I) varie entre 0,001 et 20 % en poids par rapport au poids total de la composition tinctoriale, plus particulièrement entre 0,01 à 10 % en poids,.

18. Composition selon l'une quelconque des revendications 16 ou 17 **caractérisée en ce qu'**elle comprend au moins un colorant direct additionnel, au moins une base d'oxydation éventuellement associée à au moins un coupleur, ou leurs mélanges.

19. Composition selon la revendication précédente, **caractérisée en ce que** le colorant direct additionnel est un colorant cationique ou non ionique, choisi parmi les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane, les colorants naturels, seuls ou en mélanges.

20. Composition selon la revendication 18, **caractérisée en ce que** la base d'oxydation est choisie parmi les o-phénylènediamines, les p-phénylènediamines, les bases doubles, les o-aminophénols, les p-aminophénols, les bases hétérocycliques, leurs sels d'addition avec un acide, ainsi que leurs mélanges.

21. Composition selon la revendication 18, **caractérisée en ce que** le coupleur est choisi parmi les m-aminophénols, les m-phénylènediamines, les m-diphénols, les naphtols, les coupleurs hétérocycliques, leurs sels d'addition avec un acide, ainsi que leurs mélanges.

22. Composition selon l'une quelconque des revendications 16 à 21, **caractérisée en ce qu'**elle comprend au moins un agent oxydant.

23. Procédé de coloration de fibres kératiniques consistant à mettre en contact une composition selon l'une quelconque des revendications 16 à 22, avec lesdites fibres, sèches ou humides, pendant une durée suffisante pour obtenir l'effet souhaité.

24. Dispositif à plusieurs compartiments dans lequel un premier compartiment renferme la composition selon l'une quelconque des revendications 16 à 21 et un deuxième compartiment renfermant une composition oxydante.
